(19) Europäisches Patentamt
European Patent Office
Office européen des brevets

(11) EP 3 107 893 B1

(12) # EUROPÄISCHE PATENTSCHRIFT

(45) Veröffentlichungstag und Bekanntmachung des Hinweises auf die Patenterteilung:
**25.03.2020 Patentblatt 2020/13**

(21) Anmeldenummer: **15705500.5**

(22) Anmeldetag: **18.02.2015**

(51) Int Cl.:
***C07C 217/28*** (2006.01) ***C07C 43/12*** (2006.01)
***C07C 229/12*** (2006.01)

(86) Internationale Anmeldenummer:
**PCT/EP2015/000352**

(87) Internationale Veröffentlichungsnummer:
**WO 2015/124290 (27.08.2015 Gazette 2015/34)**

(54) **FLUORTENSIDE**

FLUORINATED TENSIDES

TENSIOACTIFS FLUORÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.02.2014 EP 14000616**

(43) Veröffentlichungstag der Anmeldung:
**28.12.2016 Patentblatt 2016/52**

(73) Patentinhaber: **Merck Patent GmbH**
**64293 Darmstadt (DE)**

(72) Erfinder:
- **FRIEDRICH, Reiner**
  **64342 Seeheim-Jugenheim (DE)**
- **OSTHOFF, Julian**
  **64807 Dieburg (DE)**

(56) Entgegenhaltungen:

| | |
|---|---|
| **EP-A1- 1 522 536** | **WO-A1-2009/020907** |
| **WO-A2-2010/002623** | **JP-A- 2007 238 583** |
| **US-A1- 2012 277 460** | **US-A1- 2012 329 976** |

EP 3 107 893 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Gegenstand der vorliegenden Erfindung sind neue Verbindungen mit fluorierten Endgruppen, deren Verwendung als oberflächenaktive Substanzen und Mittel enthaltend diese Verbindungen.

Fluorhaltige Tenside besitzen aufgrund ihrer speziellen Oberflächenaktivität einzigartige Anwendungseigenschaften. Fluortenside, deren statische Oberflächenspannung sehr niedrig ist (16-20 mN/m), sind in verschiedensten Anwendungen einsetzbar und tragen z.B. zur verbesserten Benetzung von Oberflächen bei. So werden sie z.B. als Grenzflächenvermittler bzw. Emulgator oder Viskositätsminderer in Farben, Lacken oder Klebstoffen verwendet.

Klassische Fluortenside sind aus langkettigen, perfluorierten Alkylketten (C6-C8) aufgebaut und gelten potentiell als bioakkumulativ und toxisch. In der Regel enthalten Fluortenside jedoch Perfluoralkylsubstituenten, die in der Umwelt durch biologische und andere Oxidationsprozesse zu Perfluoralkancarbonsäuren und -sulfonsäuren abgebaut werden. Diese gelten als persistent und stehen z. T. im Verdacht gesundheitliche Schäden zu verursachen (G. L. Kennedy, Jr., J. L. Butenhoff, G. W. Olsen, J. C. O'Connor, A. M. Seacat, R. G. Perkins, L. B. Biegel, S. R. Murphy, D. G. Farrar, Critical Reviews in Toxicology 2004, 34, 351-384). Längerkettige Perfluoralkancarbonsäuren und -sulfonsäuren reichern sich zudem in der Nahrungskette an. Kürzerkettige Fluorbausteine sind von ihrem ökotoxikologischem Profile günstiger, zeigen jedoch in ihren Anwendungsbereichen oft schlechtere Eigenschaften.

In WO 03/010128 werden Perfluoralkyl-substituierte Amine, Säuren, Aminosäuren und Thioethersäuren beschrieben, die eine $C_{3-20}$-Perfluoralkyl-Gruppe aufweisen. Aus JP-A-2001/133984 sind oberflächenaktive Verbindungen mit Perfluoralkoxy-Ketten bekannt, die sich zum Einsatz in Antireflex-Beschichtungen eignen. Aus JP-A-09/111286 ist die Verwendung von Perfluorpolyethertensiden in Emulsionen bekannt. In WO 2006/072401 und WO 2010/003567 werden oberflächenaktive Verbindungen mit Trifluormethoxygruppen beschrieben. Verbindungen mit speziellen Fluoralkylgruppen sind in US 7,635,789, US 2008/0093582, JP 2004-18394 und WO 2010/002623 beschrieben. Teilfluorierte Verbindungen sind in US 7,737,307, EP 1 522 536 und WO 2010/002622 beschrieben. WO 2009/020907 beschreibt nicht-ionische Tenside mit einer fluorierten Gruppe.

Spezielle Anwendungen von Sulfosuccinaten und/oder Sulfotricarballylaten mit verschiedenen fluorierten Seitenketten sind in US 4,968,599 und US 4,988,610 sowie US 6, 890, 608 beschrieben und in A.R. Pitt et al., Colloids and Surfaces A: Physicochemical and Engineering Aspects, 1996, 114, 321-335; A.R. Pitt, Progr. Colloid Polym. Sci, 1997, 103, 307-317 und Z.-T. Liu et al., Ind. Eng. Chem. Res. 2007, 46, 22-28. Weitere Fluortenside, insbesondere Succinate und Tricarballylate mit fluorierten Alkylgruppen, sind in WO 2009/149807, WO 2010/003567, WO 2010/149262, WO 2011/082770 und WO 2012/084118 beschrieben.

Weiterhin besteht Bedarf nach alternativen oberflächenaktiven Substanzen, die vorzugsweise beim Abbau nicht zu langkettigen persistenten Verbindungen abbauen.

Es wurden nun neue Verbindungen gefunden, die als oberflächenaktive Substanzen geeignet sind und bevorzugt einen oder mehrere der o.g. Nachteile nicht aufweisen.

Ein erster Gegenstand der vorliegenden Erfindung sind Verbindungen der Formel (I), (IIa) oder (IIb)

$$Z_nSpacerX_x \qquad (I)$$

wobei

$Z = R_f\text{-CHF-CF}_2\text{-Y-(CH}_2)_m\text{-}$ oder
$R_f\text{-O-CHF-CF}_2\text{-Y-(CH}_2)_m\text{-}$ oder
$R_f\text{-O-(CF}_2)_{1\text{-}4}\text{-CHF-CF}_2\text{-Y-(CH}_2)_m\text{-}$ oder
$R_f\text{-O-(CF}_2)_{1\text{-}4}\text{-O-CHF-CF}_2\text{-Y-(CH}_2)_m\text{-}$ oder
$R_f\text{-O-(CF(CF}_3)\text{-CF}_2)_{1\text{-}4}\text{-CHF-CF}_2\text{-Y-(CH}_2)_m\text{-}$ oder
$R_f\text{-O-(CF(CF}_3)\text{-CF}_2)_{1\text{-}4}\text{-O-CHF-CF}_2\text{-Y-(CH}_2)_m\text{-}$ oder
$R_f\text{-O-(CF}_2\text{-O)}_{1\text{-}4}\text{-CHF-CF}_2\text{-Y-(CH}_2)_m\text{-}$ oder
$R_f\text{-O-(CF}_2\text{-CF}_2\text{-O)}_{1\text{-}4}\text{-CHF-CF}_2\text{-Y-(CH}_2)_m\text{-}$ oder
$R_f\text{-O-(CF}_2\text{-O)}_{1\text{-}4}\text{-(CF}_2\text{-CF}_2\text{-O)}_{1\text{-}4}\text{-CHF-CF}_2\text{-Y-(CH}_2)_m$
n = 2-3,
$R_f = CF_3\text{-}, CF_3\text{-CF}_2\text{-}$ oder $CF_3\text{-CF}_2\text{-CF}_2\text{-}$,
Y = O oder S, bevorzugt O,
m = 0 oder 1,

Spacer = eine gesättigte, verzweigte oder unverzweigte Kohlenwasserstoffeinheit, wobei ein oder mehrere nicht benachbarte C-Atome durch O oder N ersetzt sein können

X eine der Gruppen $-SO_3^-$, $-OSO_3^-$, $-COO^-$, $-PO_3^{2-}$, $OPO_3^{2-}$, Polyethylen- oder Polypropylenglykole, $-CH(OH)\text{-}CH_2\text{-NH-Sach}$, $-Y\text{-(CH}_2\text{-CH}_2\text{-O)}_v\text{-R}^4$, Betaine oder Sulfobetaine ist, mit Sach = verschiedene Zucker und Y =

S, O oder NH, bevorzugt O, $R^4$ = H oder Alkyl, bevorzugt H oder $CH_3$, und v = 1-100, bevorzugt 1-30 ist, x = 1,

$[F(CF_2)_{n'}(O)_o CHF\text{-}CF_2\text{-}Y\text{-}(CH_2)_m]_2$Spacer-X Formel (IIa)

$[F(CF_2)_{n'}(O)_o\text{-}(CF_2)_{1\text{-}4}\text{-}O\text{-}CHF\text{-}CF_2\text{-}Y\text{-}(CH_2)_m]_2$Spacer-X Formel (IIb)

mit n' = 1-6, m = 0 oder 1, o = 0 oder 1,
Y = O oder S, und die Gruppierung (Spacer-X) = $CR^5(CH_2)_{n''}OH$, $CR^5(CH_2)_{n''}SH$, $CR^5(CH_2)_{n''}COOH$, $CR^5(CH_2)_{n''}SO_3H$, $CR^5(CH_2)_{n''}NH_2$, $CR^5(CH_2)_{n''}NR'_2$, $CR^5(CH_2)_{n''}N^+(CH_3)_3Cl^-$, $CR^5(CH_2)_{n''}NR'_2\text{-}CH_2\text{-}COO^-$, $CR^5(CH_2)_{n''}O(CHR^a\text{-}CHR^bO)_nR''$, $CR^5(CH_2)_{n''}S(CHR^a\text{-}CHR^bO)_nR''$ oder $CR^5(CH_2)_{n''}NH(CHR^a\text{-}CHR^bO)_nR''$, n" = 0 oder 1, n = 1-30, und $R^5$, R', R", $R^a$ und $R^b$ unabhängig voneinander = H oder Alkyl
und wobei alle Indizes so gewählt sind, dass keine O-O Bindungen vorliegen.

Wenn X = $-SO_3H$ oder $-SO_3^-$ ist, dann ist Z bevorzugt nicht über eine -OCO-Bindung an den Spacer gebunden.

Bei den Kohlenwasserstoff-Einheiten des Spacers der Verbindungen der Formel (I) kann es sich um aliphatische handeln. Vorzugsweise ist die Gruppe Z in den oberflächenaktiven Verbindungen dabei an eine gesättigte, verzweigte oder unverzweigte Alkylengruppe, wobei ein oder mehrere nicht benachbarte C-Atome durch O oder N, bevorzugt O, ersetzt sein können, gebunden. Bevorzugt sind z. B. C1-C6-Alkylengruppen, insbesondere C1-C4-Alkylengruppen. In einer Erfindungsvariante wird als bevorzugte Heteroatome enthaltende Kohlenwasserstoffeinheit eine Polyethylen- oder Polypropylenglykoleinheit verwendet.

Die Gruppe Z kommt in der oberflächenaktiven Verbindung mehrfach vor, zweimal oder dreimal. In einer Erfindungsvariante können Verbindungen der Formel (I) verschiedene Gruppe Z enthalten.

In den Verbindungen der Formel (I) ist n = 2-3 und x = 1.

In den Verbindungen der Formel (I) ist Z gleich:

$R_f\text{-}CHF\text{-}CF_2\text{-}Y\text{-}(CH_2)_m$- oder
$R_f\text{-}O\text{-}CHF\text{-}CF_2\text{-}Y\text{-}(CH_2)_m$- oder
$R_f\text{-}O\text{-}(CF_2)_{1\text{-}4}\text{-}CHF\text{-}CF_2\text{-}Y\text{-}(CH_2)_m$- oder
$R_f\text{-}O\text{-}(CF_2)_{1\text{-}4}\text{-}O\text{-}CHF\text{-}CF_2\text{-}Y\text{-}(CH_2)_m$- oder
$R_f\text{-}O\text{-}(CF(CF_3)\text{-}CF_2)_{1\text{-}4}\text{-}CHF\text{-}CF_2\text{-}Y\text{-}(CH_2)_m$- oder
$R_f\text{-}O\text{-}(CF(CF_3)\text{-}CF_2)_{1\text{-}4}\text{-}O\text{-}CHF\text{-}CF_2\text{-}Y\text{-}(CH_2)_m$- oder
$R_f\text{-}O\text{-}(CF_2\text{-}O)_{1\text{-}4}\text{-}CHF\text{-}CF_2\text{-}Y\text{-}(CH_2)_m$- oder
$R_f\text{-}O\text{-}(CF_2\text{-}CF_2\text{-}O)_{1\text{-}4}\text{-}CHF\text{-}CF_2\text{-}Y\text{-}(CH_2)_m$- oder
$R_f\text{-}O\text{-}(CF_2\text{-}O)_{1\text{-}4}\text{-}(CF_2\text{-}CF_2\text{-}O)_{1\text{-}4}\text{-}CHF\text{-}CF_2\text{-}Y\text{-}(CH_2)_m$- ist mit m = 0 oder 1.

Insbesondere bevorzugt sind Verbindungen der Formel (I) in denen Z gleich:

$R_f\text{-}CHF\text{-}CF_2\text{-}Y\text{-}(CH_2)_m$- oder
$R_f\text{-}O\text{-}CHF\text{-}CF_2\text{-}Y\text{-}(CH_2)_m$- oder
$R_f\text{-}O\text{-}(CF_2)_{1\text{-}4}\text{-}CHF\text{-}CF_2\text{-}Y\text{-}(CH_2)_m$- oder
$R_f\text{-}O\text{-}(CF_2)_{1\text{-}4}\text{-}O\text{-}CHF\text{-}CF_2\text{-}Y\text{-}(CH_2)_m$- ist.

Vor allem Verbindungen der Formel (I) mit Z = $R_f\text{-}O\text{-}CHF\text{-}CF_2\text{-}Y\text{-}(CH_2)_m$- oder $R_f\text{-}O\text{-}(CF_2)_{1\text{-}4}\text{-}O\text{-}CHF\text{-}CF_2\text{-}Y\text{-}(CH_2)_m$- sind bevorzugt.

In den Verbindungen der Formel (I) ist Rf gleich:
$CF_3$- oder $CF_3\text{-}CF_2$- oder $CF_3\text{-}CF_2\text{-}CF_2$- ist.

Im Besonderen sind Verbindungen der Formel (I) bevorzugt, in denen n = 2-3, x = 1, der Spacer eine gesättigte, verzweigte oder unverzweigte Kohlenwasserstoff-Einheit ist, Rf gleich $CF_3$-, $CF_3\text{-}CF_2$- oder $CF_3\text{-}CF_2\text{-}CF_2$-ist und Z gleich:

$R_f\text{-}CHF\text{-}CF_2\text{-}Y\text{-}(CH_2)_m$- oder
$R_f\text{-}O\text{-}CHF\text{-}CF_2\text{-}Y\text{-}(CH_2)_m$- oder
$R_f\text{-}O\text{-}(CF_2)_{1\text{-}4}\text{-}CHF\text{-}CF_2\text{-}Y\text{-}(CH_2)_m$- oder
$R_f\text{-}O\text{-}(CF_2)_{1\text{-}4}\text{-}O\text{-}CHF\text{-}CF_2\text{-}Y\text{-}(CH_2)_m$- oder
$R_f\text{-}O\text{-}(CF(CF_3)\text{-}CF_2)_{1\text{-}4}\text{-}CHF\text{-}CF_2\text{-}Y\text{-}(CH_2)_m$- oder
$R_f\text{-}O\text{-}(CF(CF_3)\text{-}CF_2)_{1\text{-}4}\text{-}O\text{-}CHF\text{-}CF_2\text{-}Y\text{-}(CH_2)_m$- oder

$R_f$-O-($CF_2$-O)$_{1-4}$-CHF-$CF_2$-Y-($CH_2$)$_m$- oder
$R_f$-O-($CF_2$-$CF_2$-O)$_{1-4}$-CHF-$CF_2$-Y-($CH_2$)$_m$- oder
$R_f$-O-($CF_2$-O)$_{1-4}$-($CF_2$-$CF_2$-O)$_{1-4}$-CHF-$CF_2$-Y-($CH_2$)$_m$- ist mit m = 0 oder 1.

Insbesondere bevorzugt sind hierbei Verbindungen, in denen Z gleich:

$R_f$-CHF-$CF_2$-Y-($CH_2$)$_m$- oder
$R_f$-O-CHF-$CF_2$-Y-($CH_2$)$_m$- oder
$R_f$-O-($CF_2$)$_{1-4}$-CHF-$CF_2$-Y-($CH_2$)$_m$- oder
$R_f$-O-($CF_2$)$_{1-4}$-O-CHF-$CF_2$-Y-($CH_2$)$_m$- ist, vor allem Verbindungen mit Z = Rf-O-CHF-$CF_2$-Y-($CH_2$)$_m$- oder $R_f$-O-($CF_2$)$_{1-4}$-O-CHF-$CF_2$-Y-($CH_2$)$_m$-.

Besonders bevorzugt sind Verbindungen in denen die genannten Variablen, insbesondere alle Variablen, die bevorzugten Bedeutungen, insbesondere die besonders bevorzugten Bedeutungen, haben.

In den erfindungsgemäßen Verbindungen ist X eine der Gruppen -$SO_3^-$, -$OSO_3^-$, -$COO^-$, -$PO_3^{2-}$, $OPO_3^{2-}$, Polyethylen- oder Polypropylenglykole, -CH(OH)-$CH_2$-NH-Sach, -Y-($CH_2$-$CH_2$-O)$_v$-$R^4$, Betaine oder Sulfobetaine ist, mit Sach = verschiedene Zucker und Y = S, O oder NH, bevorzugt O, $R^4$ = H oder Alkyl, bevorzugt H oder $CH_3$, und v = 1-100, bevorzugt 1-30 ist

Zu den anionischen Gruppen gehören dabei -$COO^-$, -$SO_3^-$, -$OSO_3^-$, -$PO_3^{2-}$ und -$OPO_3^{2-}$, wobei jede einzelne dieser Gruppen für sich genommen bevorzugt sein kann.

Insbesondere eine Sulfonatgruppe -$SO_3^-$ ist bevorzugt.

Bevorzugtes Gegenion für anionische Gruppen X ist ein einwertiges Kation, insbesondere $H^+$, ein Alkalimetall-Kation oder $NR_4^+$, wobei R = H oder C1 - C6-Alkyl ist und alle R gleich oder verschieden sein können. Insbesondere bevorzugt sind $H^+$, $Na^+$, $K^+$, $Li^+$ und $NH_4^+$, besonders bevorzugt $Na^+$.

Eine nicht-ionische Gruppe X ist eine Polyethylen- oder Polypropylenglykoleinheit.

Eine weitere nicht-ionische Gruppe X ist die Gruppe -CH(OH)-$CH_2$-NH-Sach mit Sach = verschiedene Zucker und die Gruppe -Y-($CH_2$-$CH_2$-O)$_v$-$R^4$ mit Y = S, O oder NH, bevorzugt O, $R^4$ = H oder Alkyl, bevorzugt H oder $CH_3$, und v = 1-100, bevorzugt 1-30, insbesondere auch 1-25.

Eine amphotere Gruppe kann ausgewählt sein aus den funktionellen Gruppen der Betaine und der Sulfobetaine, insbesondere ausgewählt aus,:

| |
|---|
| -[(C(=O)-NH-($CH_2$)$_{(1-8)}$]$_{(0 \text{ oder } 1)}$-$N^+R^1R^2$-$CH_2$-$COO^-$, wobei $R^1$ und $R^2$ jeweils unabhängig voneinander stehen für einen C1-8-Alkylrest, vorzugsweise Methyl oder Ethyl |
| -C(=O)-NH-($CH_2$)$_{1-3}$-$N^+R^1R^2$-$CH_2$-CH(OH)-$CH_2$-(O)$_{(0 \text{ oder } 1)}$-(S oder P)$O_3^-$, wobei $R^1$ und $R^2$ jeweils unabhängig voneinander stehen für einen C1-8-Alkylrest, vorzugsweise Methyl oder Ethyl |

Besonders bevorzugte erfindungsgemäße Verbindungen sind solche, die als hydrophile Gruppe X eine der bevorzugten anionische Gruppen, der bevorzugten nicht-ionischen Gruppen oder der bevorzugten zwitterionischen Gruppen enthalten.

Die erfindungsgemäßen Verbindungen enthaltene die Gruppen -$SO_3^-$, -$OSO_3^-$, -$COO^-$, -$PO_3^{2-}$ oder $OPO_3^{2-}$, Polyethylen- oder Polypropylenglykole, -CH(OH)-$CH_2$-NH-Sach, -Y-($CH_2$-$CH_2$-O)$_v$-$R^4$, Betaine, oder Sulfobetaine enthalten. Bevorzugte Gegenionen sind hierbei $H^+$, $Na^+$, $K^+$ und $NH_4^+$, insbesondere $Na^+$. Insbesondere bevorzugt sind: -$SO_3^-$, -$COO^-$, Polyethylen- oder Polypropylenglykole, Sulfobetaine, die Gruppe -CH(OH)-$CH_2$-NH-Sach und die Gruppe -Y-($CH_2$-$CH_2$-O)$_v$-$R^4$. Hierbei ist Sach = verschiedene Zucker und Y = S, O oder NH, bevorzugt O, $R^4$ = H oder Alkyl, bevorzugt H oder $CH_3$, und v = 1-100, bevorzugt 1-30, insbesondere auch 1-25. Besonders vorteilhaft können auch Verbindungen mit X = -$SO_3^-$ sein.

Besonders vorteilhaft sind Verbindungen der Formel (I), in denen eine oder mehrere der Variablen die bevorzugten Bedeutungen haben. Besonders vorteilhaft sind Verbindungen der Formel (I), in denen alle genannten Variablen die bevorzugten Bedeutungen, insbesondere die besonders bevorzugten Bedeutungen, haben.

In einer Erfindungsvariante sind Verbindungen der Formel (I) bevorzugt, in denen die Gruppierung (Spacer-X) eine der folgenden Bedeutungen hat: $CR^5(CH_2)_{n''}OH$, $CR^5(CH_2)_{n''}SH$, $CR^5(CH_2)_{n''}COOH$, $CR^5(CH_2)_{n''}SO_3H$, $CR^5(CH_2)_{n''}NH_2$, $CR^5(CH_2)_{n''}NR'_2$, $CR^5(CH_2)_{n''}N^+(CH_3)_3Cl^-$, $CR^5(CH_2)_{n''}NR'_2$-$CH_2$-$COO^-$, $CR^5(CH_2)_{n''}O(CHR^a\text{-}CHR^bO)_nR''$, $CR^5(CH_2)_{n''}S(CHR^a\text{-}CHR^bO)_nR''$ oder $CR^5(CH_2)_{n''}NH(CHR^a\text{-}CHR^bO)_nR''$ ist, mit n = 1-30, bevorzugt = 1-25, insbesondere 4-25, $R^5$, R', R", $R^a$ und $R^b$ unabhängig voneinander = H oder Alkyl, bevorzugt H oder C1-C4 Alkyl. $R^5$ ist bevorzugt H oder $CH_3$, insbesondere H.

Insbesondere bevorzugt sind auch Verbindungen der Formel (I), in denen diese Gruppierungen (Spacer-X)

vorliegen, vor allem die bevorzugten Gruppierung, in denen n = 2-3, bevorzugt = 2, x = 1, und Rf gleich $CF_3$-, $CF_3$-$CF_2$- oder $CF_3$-$CF_2$-$CF_2$- ist und Z gleich:

$R_f$-CHF-$CF_2$-Y-$(CH_2)_m$- oder
$R_f$-O-CHF-$CF_2$-Y-$(CH_2)_m$- oder
$R_f$-O-$(CF_2)_{1-4}$-CHF-$CF_2$-Y-$(CH_2)_m$- oder
$R_f$-O-$(CF_2)_{1-4}$-O-CHF-$CF_2$-Y-$(CH_2)_m$- oder
$R_f$-O-$(CF(CF_3)$-$CF_2)_{1-4}$-CHF-$CF_2$-Y-$(CH_2)_m$- oder
$R_f$-O-$(CF(CF_3)$-$CF_2)_{1-4}$-O-CHF-$CF_2$-Y-$(CH_2)_m$- oder
$R_f$-O-$(CF_2$-$O)_{1-4}$-CHF-$CF_2$-Y-$(CH_2)_m$- oder
$R_f$-O-$(CF_2$-$CF_2$-$O)_{1-4}$-CHF-$CF_2$-Y-$(CH_2)_m$- oder
$R_f$-O-$(CF_2$-$O)_{1-4}$-$(CF_2$-$CF_2$-$O)_{1-4}$-CHF-$CF_2$-Y-$(CH_2)_m$- ist mit m = 0 oder 1.

Insbesondere bevorzugt sind hierbei Verbindungen, in denen Z gleich:

$R_f$-CHF-$CF_2$-Y-$(CH_2)_m$- oder
$R_f$-O-CHF-$CF_2$-Y-$(CH_2)_m$- oder
$R_f$-O-$(CF_2)_{1-4}$-CHF-$CF_2$-Y-$(CH_2)_m$- oder
$R_f$-O-$(CF_2)_{1-4}$-O-CHF-$CF_2$-Y-$(CH_2)_m$- ist, vor allem Verbindungen mit Z = Rf-O-CHF-$CF_2$-Y-$(CH_2)_m$- oder $R_f$-O-$(CF_2)_{1-4}$-O-CHF-$CF_2$-Y-$(CH_2)_m$-.

Besonders bevorzugte Verbindungen der Erfindung sind Verbindungen der Formeln (IIa) und (IIb):

$[F(CF_2)_{n'}(O)_o CHF$-$CF_2$-Y-$(CH_2)_m]_2$Spacer-X    Formel (IIa)

$[F(CF_2)_{n'}(O)_o$-$(CF_2)_{1-4}$-O-CHF-$CF_2$-Y-$(CH_2)_m]_2$Spacer-X    Formel (IIb)

mit n' = 1-6, bevorzugt 1-3, m = 0 oder 1, o = 0 oder 1, bevorzugt 1, Y = O oder S, bevorzugt O, und die Gruppierung (Spacer-X) = $CR^5(CH_2)_{n''}OH$, $CR^5(CH_2)_{n''}SH$, $CR^5(CH_2)_{n''}COOH$, $CR^5(CH_2)_{n''}SO_3H$, $CR^5(CH_2)_{n''}NH_2$, $CR^5(CH_2)_{n''}NR'_2$, $CR^5(CH_2)_{n''}N^+(CH_3)_3Cl^-$, $CR^5(CH_2)_{n''}NR'_2$-$CH_2$-$COO^-$, $CR^5(CH_2)_{n''}O(CHR^a$-$CHR^bO)_nR''$, $CR^5(CH_2)_{n''}S(CHR^a$-$CHR^bO)_nR''$ oder $CR^5(CH_2)_{n''}NH(CHR^a$-$CHR^bO)_nR''$ ist, mit n" = 0 oder 1, n = 1-30, bevorzugt = 1-25, insbesondere 4-25, $R^5$, R', R", $R^a$ und $R^b$ unabhängig voneinander = H oder Alkyl, bevorzugt H oder C1-C4 Alkyl. $R^5$ ist bevorzugt H oder $CH_3$, insbesondere H.

Insbesondere bevorzugt sind Verbindungen der Formeln (IIa) und (IIb) in denen eine oder mehrere der Variablen die bevorzugten Bedeutungen. haben. Besonders vorteilhaft sind Verbindungen der Formeln (IIa) und (IIb), in denen alle genannten Variablen die bevorzugten Bedeutungen haben.

Bevorzugt sind vor allem Verbindungen der Formeln (III), (III'), (IV) und (IV') in denen die Variablen die für die Formel (I) angegebenen Bedeutungen haben, insbesondere die bevorzugten Bedeutungen:

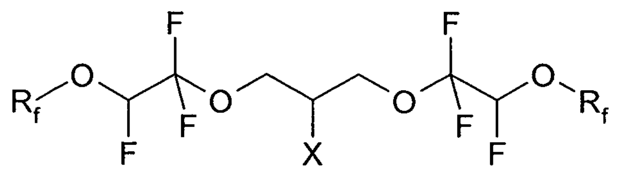
Formel (III)

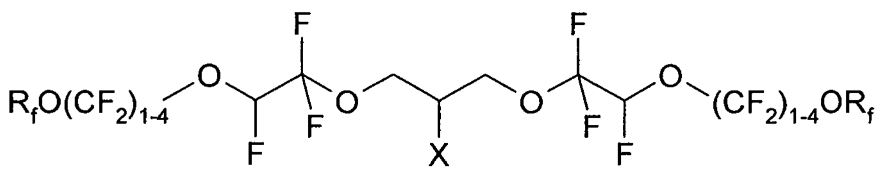

Formel (III')

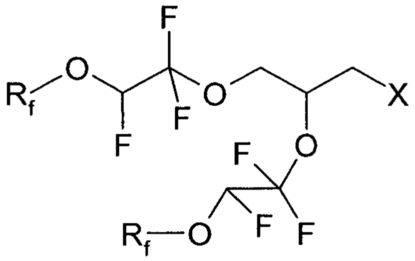

Formel (IV)

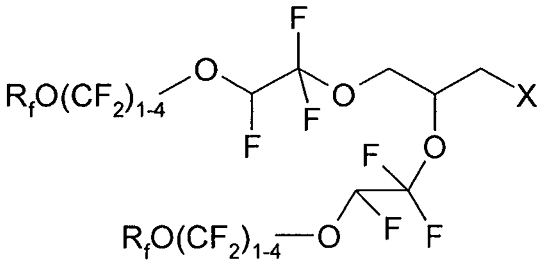

Formel (IV')

In einer Erfindungsvariante sind Verbindungen der Formeln (III) und (IV) bevorzugt, in denen X eine hydrophile Gruppe ist, wie sie in der Gruppierung (Spacer-X) in den Formeln (IIa) und (IIb) genannt ist, insbesondere solche Verbindungen, in denen Rf gleich $CF_3$-, $CF_3$-$CF_2$- oder $CF_3$-$CF_2$-$CF_2$- ist.

In einer weiteren Erfindungsvariante sind Verbindungen der Formeln (III') und (IV') bevorzugt, in denen X eine hydrophile Gruppe ist, wie sie in der Gruppierung (Spacer-X) in den Formeln (IIa) und (IIb) genannt ist, insbesondere solche Verbindungen, in denen Rf gleich $CF_3$-, $CF_3$-$CF_2$- oder CF3-CF2-CF2- ist.

Bevorzugt sind auch Verbindungen der Formeln (IIIa), (III'a), (IIIb), (III'b), (IVa), (IV'a), (IVb), (IV'b), (IVc) und (IV'c), in denen die Variablen die für die Formeln (I) und (IIa) und (IIb) angegebenen Bedeutungen haben, insbesondere die bevorzugten Bedeutungen, und PEG für Polyethylenglycol, Polypropylenglycol, Polyethylenglycolalkylether oder Polypropylenglycolalkylether steht. Alkylether sind bevorzugt C1-C4-Alkylether, insbesondere C1-C2-Alkylether, vor allem Methylether.

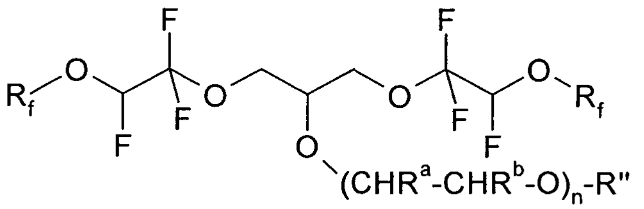

Formel (IIIa)

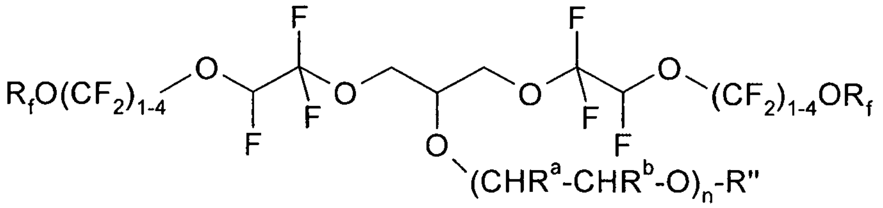

Formel (III'a)

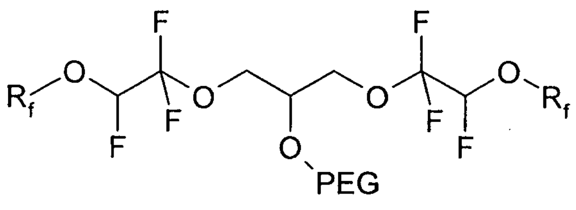

Formel (IIIb)

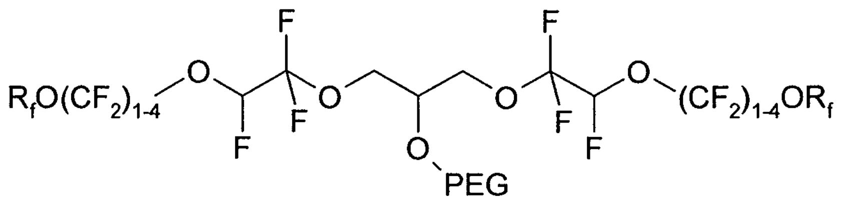

Formel (III'b)

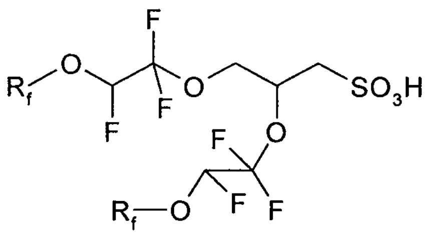

Formel (IVa)

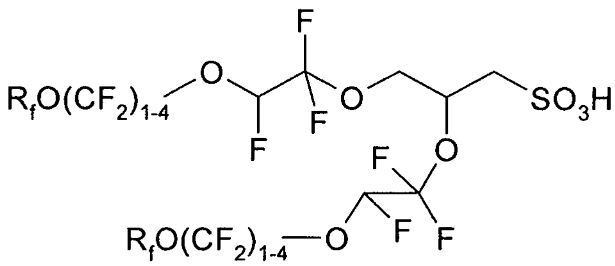

Formel (IV'a)

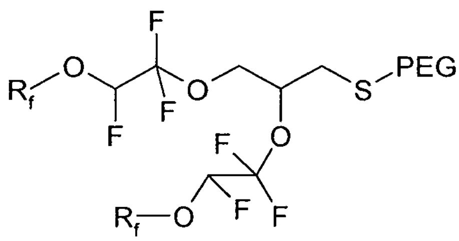

Formel (IVb)

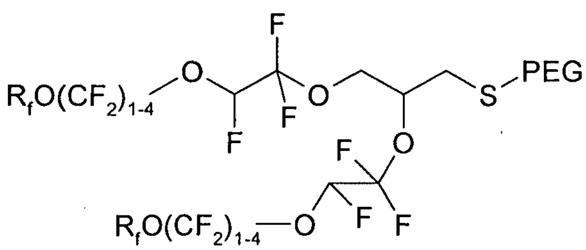

Formel (IV'b)

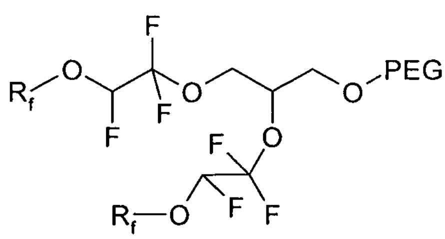

Formel (IVc)

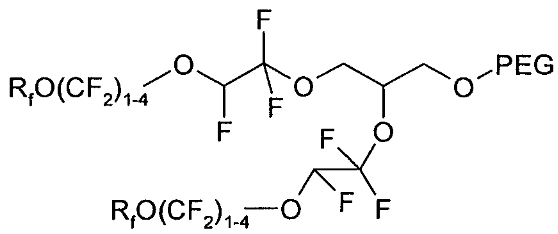

Formel (IV'c)

Insbesondere die folgenden Verbindungen der Formeln (V) bis (XII) sind besonders bevorzugt:

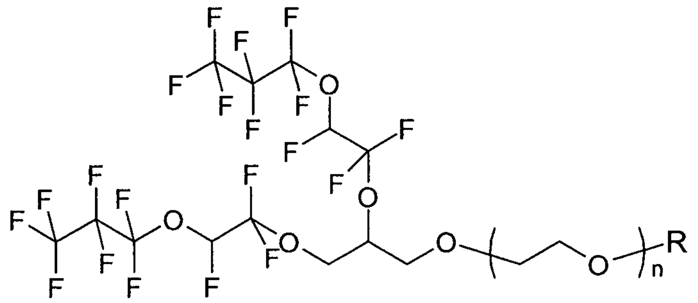

n~6 und R=H oder Methyl

Formel (V)

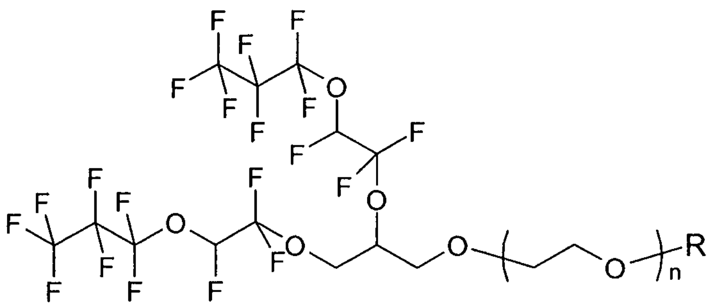

n~12 und R=H oder Methyl

Formel (VI)

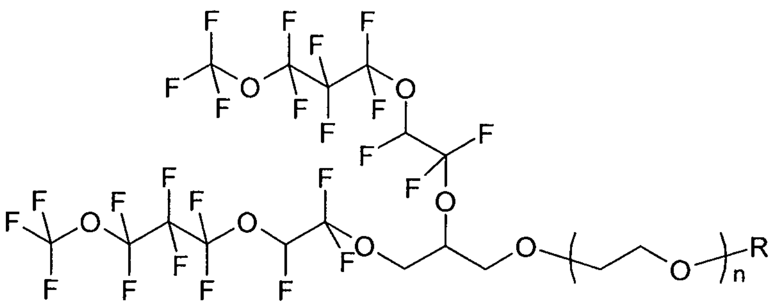

n~12 und R=H oder Methyl

Formel (VII)

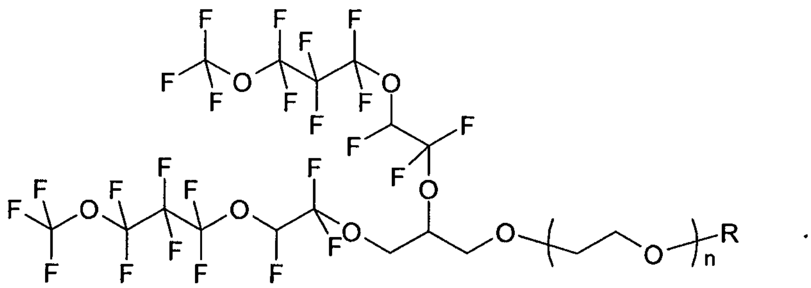

n~24 und R=H oder Methyl Formel (VIII)

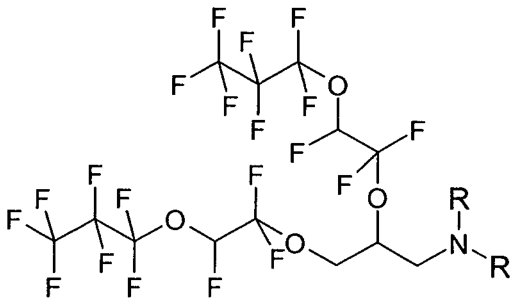

R=Methyl oder Ethyl Formel (IX)

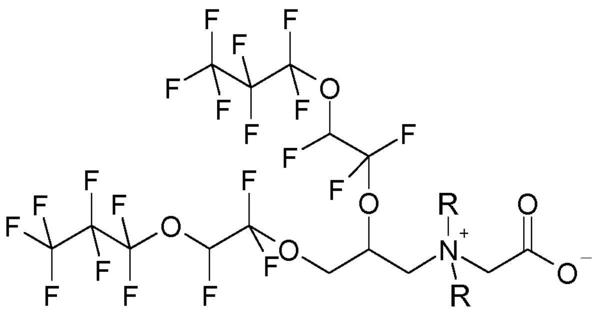

R=Methyl oder Ethyl Formel (X)

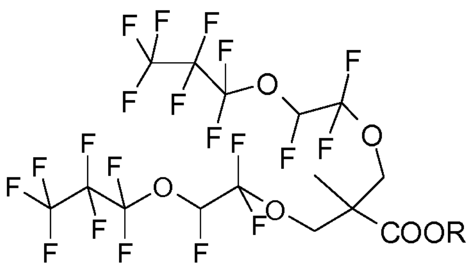

R=H, Methyl oder Ethyl Formel (XI)

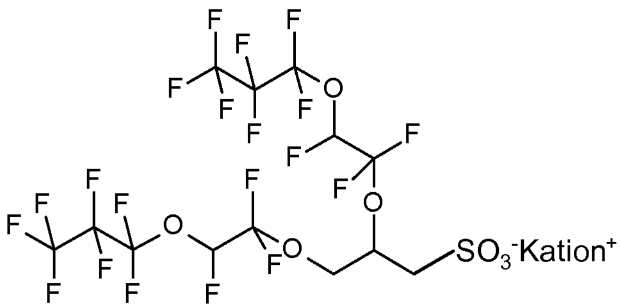

Kation$^+$ = $Na^+$, $K^+$ oder $NH_4^+$ Formel (XII)

Die erfindungsgemäßen Verbindungen sind nach dem Fachmann bekannten Verfahren herstellbar. Die folgenden Schemata zeigen Beispielsynthesen für erfindungsgemäße Verbindungen. Diese Verfahren sind dem Fachmann generell bekannt und können unter üblichen Bedingungen durchgeführt werden.

Die erfindungsgemäßen Verbindungen, insbesondere Verbindungen der Formeln (IIa) und (IIb), bevorzugt Verbindungen der Formeln (III), (III'), (IV) und (IV'), können bevorzugt nach den folgenden Syntheserouten (beispielhaft dargestellt für Verbindungen mit Rf = $C_4F_9$) hergestellt werden. Besonders bevorzugt sind hierbei Verbindungen der Formeln (IIIa), (IIIb), (IVa), (IVb), (IVc), (III'a), (III'b), (IV'a), (IV'b) und (IV'c), insbesondere der Formeln (V) bis (XII).

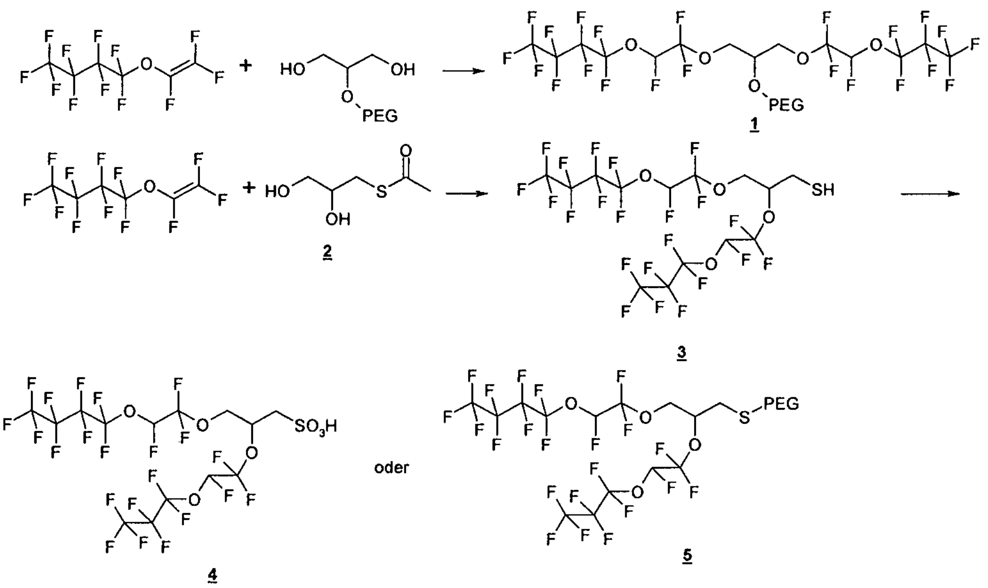

1 2 3 4 oder 5

Trifunktionelle Verbindungen müssen dabei so geschützt/aktiviert werden, dass nur zwei Perfluorolefinmoleküle mit dem Molekül reagieren können. Dies lässt sich durch bekannte Schutzgruppen und entsprechende Syntheseabfolge realisieren. Verbindungen wie **1** lassen sich bevorzugt durch Umsetzung eines PEG-ylierten Glycerins (ein mit PEG umgesetztes Glycerin)herstellen.

Der entsprechende Thioether **5** ist über das geschützte Thioglycerin **2** zugänglich. Eine Zwischenstufe ist dabei Verbindung **3,** die auch durch Oxidation zum entsprechenden Sulfonat **4** umgesetzt werden kann.

Bei Verwendung von Serinol geht man an bestem von dessen Oxalatsalz aus oder der tert-Butyloxycarbonyl (BOC) geschützten Verbindung aus. Verbindungen wie **7** lassen sich bevorzugt aus einem monogeschütztem Glycerin **6** herstellen.

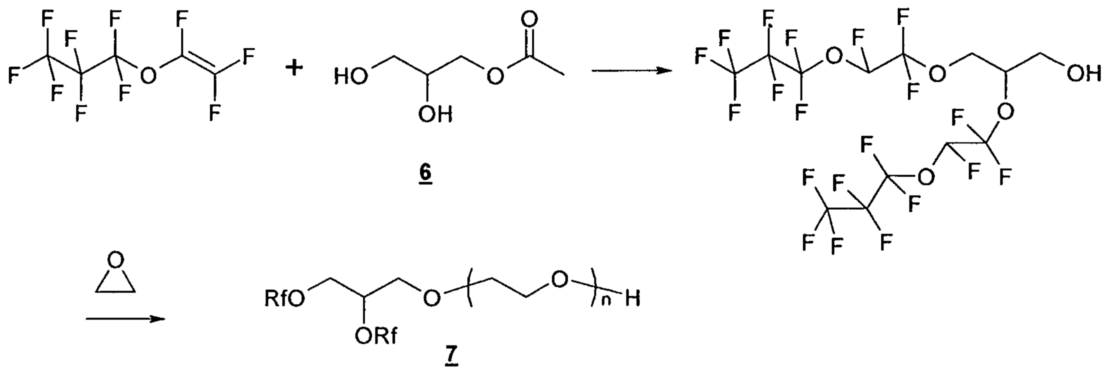

6

7

Bevorzugt können die folgenden beispielhaft genannten Perfluorolefinverbindungen eingesetzt werden:

$CF_3$-CF=$CF_2$ $CF_3$-$CF_2$-$CF_2$-OCF=$CF_2$
$CF_3$-$CF_2$-CF=$CF_2$ $CF_3$-$CF_2$-OCF=$CF_2$
$CF_3$-$CF_2$-$CF_2$-CF=$CF_2$ $CF_3$-OCF=$CF_2$

Bevorzugt können die folgenden beispielhaft genannten Alkohole eingesetzt werden:

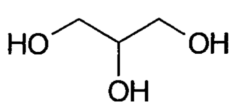 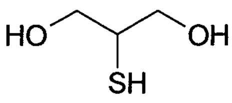 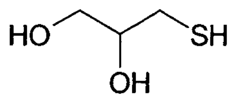

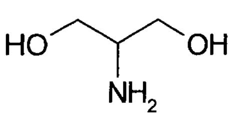 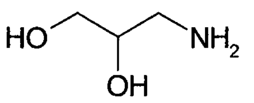 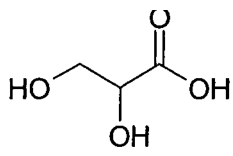

Sinnvoll kann es dabei sein die Selektivität der einzelnen funktionellen Gruppen zu schützen:

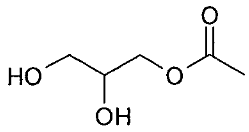 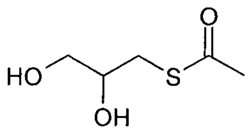 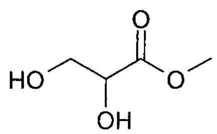 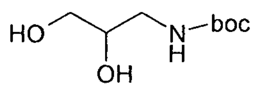

Vorteil bei diesen Verbindungen ist, dass nach der Umsetzung mit dem Perfluorolefin die Schutzgruppe durch Verkochen in wässriger Natronlauge bzw ansäuern leicht entfernt werden kann.

Die Herstellung weiterer erfindungsgemäßer Verbindungen, insbesondere weiterer Verbindungen der Formeln (IIa) und (IIb), bevorzugt weiterer Verbindungen der Formeln (III), (III'), (IV) und (IV'), insbesondere weiterer Verbindungen der Formeln (IIIa), (IIIb), (IVa), (IVb), (IVc), (III'a), (III'b), (IV'a), (IV'b) und (IVc), kann analog zu den oben gezeigten beispielhaften Reaktionen oder nach anderen dem Fachmann an sich aus der Literatur bekannten Methoden erfolgen. Besonders bevorzugt sind hierbei Verbindungen der Formeln (V) bis (XII). Die Herstellung weiterer erfindungsgemäßer Verbindungen kann auch nach anderen dem Fachmann an sich aus der Literatur bekannten Methoden erfolgen. Diese Verfahren sind dem Fachmann generell bekannt und können unter üblichen Bedingungen durchgeführt werden. Die verwendeten Alkohole sind kommerziell erhältlich und/oder ihre Herstellung ist dem Fachmann geläufig.

Vorteile der erfindungsgemäßen Verbindungen können insbesondere sein:

- eine Oberflächenaktivität, die der konventioneller Kohlenwasserstoff-Tenside hinsichtlich Effizienz und/oder Effektivität gleich oder überlegen ist,
- biologische und/oder abiotische Abbaubarkeit der Substanzen ohne Bildung persistenter perfluorierter Abbauprodukte wie PFOA (Perfluoroctansäure) oder PFOS (Perfluoroctansulfonat),

- nach einfachen Verfahren herstellbar,
- schwache Schaumwirkung und/oder geringe Schaumstabilisierung,
- gute Verarbeitbarkeit in Formulierungen und/oder
- Lagerstabilität.

Bevorzugt können die erfindungsgemäßen Verbindungen eine besondere Oberflächenaktivität aufweisen. Die erfindungsgemäßen Verbindungen der Formel (I), insbesondere die Verbindungen der Formeln (IIa) und (IIb) und bevorzugt der Formeln (III), (III'), (IV) und (IV'), insbesondere der Formeln (lila), (IIIb), (IVa), (IVb), (IVc), (III'a), (III'b), (IV'a), (IV'b) und (IVc), besonders bevorzugt der Formeln (V) -(XII), können außerdem gegenüber den Fluortensiden des Standes der Technik deutlich verbesserte Umwelteigenschaften aufweisen, da sie weder chemisch noch biologisch zu langkettigen PFCAs oder PFASs abbauen.

Bevorzugt können die erfindungsgemäßen Verbindungen durch entsprechende Umwelteinflüsse vollständig in mineralisierbare/regenerierbare Verbindungen überführt werden.

Der Abbau der erfindungsgemäßen Verbindungen kann bevorzugt nach zwei Mechanismen erfolgen. Im ersten Schritt kann durch biologische Aktivität das Kohlenstoffgerüst soweit abgebaut werden, dass (teil)fluorierte Verbindungen entstehen, die nicht toxisch sind und einen hohen Dampfdruck besitzen. Aufgrund der hohen Flüchtigkeit, können diese Verbindungen in die Atmosphäre gelangen und in der Stratosphäre durch die dort vorherrschende intensive UV-Strahlung zu niedermolekularen Verbindungen (HF, $COF_2$ etc.) zersetzt werden. Diese Zersetzungsprodukte können dann mit dem Regen aus der Atmosphäre ausgewaschen, in den Boden überführt und dort mineralisiert werden.

Um diesen Zyklus durchlaufen zu können ist es bevorzugt, dass die Endprodukte bei der biologischen Zersetzung nicht perfluoriert sind und keine (stabilen) Salze entstehen können.

Ein weiterer Gegenstand der Erfindung ist ein Verfahren zum Abbau von fluorhaltigen Verbindungen umfassend die folgenden Schritte:

a) biologische und/oder abiotischer Abbau des Kohlenstoffgerüsts der fluorhaltigen Verbindungen unter Bildung von, vorzugsweise nicht toxischen, fluorhaltigen Verbindungen mit einem ausreichend hohen Dampfdruck,
b) Überführen der in Schritt a) gebildeten fluorhaltigen Verbindungen mit hohem Dampfdruck in eine Gasphase,
c) Abbau der in Schritt a) gebildeten fluorhaltigen Verbindungen mit hohem Dampfdruck zu niedermolekularen Verbindungen durch UV-Bestrahlung in der Gasphase,
d) Überführen der in Schritt c) gebildeten niedermolekularen Verbindungen aus der Gasphase in eine flüssige und/oder feste Phase,
e) Mineralisierung der Schritt c) gebildeten niedermolekularen Verbindungen der flüssigen und/oder festen Phase.

Bevorzugt werden in Schritt a) keine fluorhaltigen, Salze gebildet. Insbesondere werden in Schritt a) keine perfluorierten Verbindungen gebildet.

Bevorzugt werden fluorhaltige Tenside dem beschriebenen Abbauverfahren unterzogen, insbesondere solche Tenside, die auf teilfluorierten beruhen.

In dem genannten Abbauverfahren werden bevorzugt Verbindungen der Formel (I), bevorzugt der Formeln (IIa) und (IIb), insbesondere der Formeln (III), (III'), (IV) und (IV') eingesetzt. Besonders bevorzugt sind hierbei Verbindungen der Formeln (IIIa), (IIIb), (IVa), (IVb), (IVc), (III'a), (III'b), (IV'a), (IV'b) und (IV'c), insbesondere solche der Formeln (V) bis (XII).

Ein weiterer Gegenstand der vorliegenden Erfindung ist die Verwendung der erfindungsgemäßen Verbindungen und den vorstehend beschriebenen bevorzugten Ausführungsformen als oberflächenaktive Mittel, beispielsweise zur Verbesserung des Verlaufsverhaltens und des Benetzungsvermögens von Coatingformulierungen. Fluortenside der Formeln (IIa) und (IIb), insbesondere der Formeln (III), (III'), (IV) und (IV'), werden bevorzugt verwendet, insbesondere die genannten besonders bevorzugten Verbindungen. Bevorzugt sind vor allem Verbindungen der Formeln (IIIa), (IIIb), (IVa), (IVb), (IVc), (III'a), (III'b), (IV'a), (IV'b) und (IV'c), insbesondere Verbindungen der Formeln (V) bis (XII).

Neben den Verbindungen der Formel (I), insbesondere der bevorzugten Verbindungen der Formeln (IIa), (IIb), (III), (III'), (IV), (IV'), (IIIa), (IIIb), (IVa), (IVb), (IVc), (III'a), (III'b), (IV'a), (IV'b) und (IV'c), vor allem der Formeln (V)-(XII), können die erfindungsgemäßen Mischungen auch Lösemittel, Additive, Hilfs- und Füllstoffe sowie nicht fluorierte Tenside enthalten. Beispielhaft seien genannt Silikonpartikel, Weichmacher und oberflächenmodifizierte Pigmente.

Bevorzugte Einsatzgebiete sind beispielsweise die Verwendung der erfindungsgemäßen Fluortenside der Formel (I) und der bevorzugten Verbindungen der Formeln (IIa), (IIb), (III), (III'), (IV), (IV'), (IIIa), (IIIb), (IVa), (IVb), (IVc), (III'a), (III'b), (IV'a), (IV'b) und (IV'c), vor allem der Formeln (V)-(XII), als Additive in Zubereitungen zur Oberflächenbeschichtung, wie Farben, Lacken, Schutzanstrichen, Spezialcoatings in elektronischen oder Halbleiter-Anwendungen (z.B. Photolacken, Top Antireflective Coatings, Bottom Antireflective Coatings) oder in optischen Anwendungen (z.B. photographischen Beschichtungen, Beschichtungen optischer Elemente), in Agrochemikalien, in Poliermitteln und Wachsen, z.B. für Mobiliar, Fußböden und Automobile, insbesondere in Bodenpolituren, in Feuerlöschmitteln, Schmierstoffen,

in photolithographischen Verfahren, insbesondere in Immersionsphotolithographie-Verfahren, z.B. in Entwicklerlösungen, Spüllösungen, Immersionsölen und/oder in den Photoresists selbst, vor allem zur Herstellung von gedruckten Schaltungen oder in Additivzubereitungen zur Additivierung entsprechender Zubereitungen.

Darüber hinaus eignen sich die erfindungsgemäß als Tensid verwendbaren Verbindungen für Wasch- und Reinigungsanwendungen, sowie für eine Verwendung als Additive/Tenside in kosmetischen Produkten wie z. B. Haar- und Körperpflegeprodukten (z.B. Shampoos, Haarspülungen und Haarkonditionierern), Schaumbädern, Cremes oder Lotionen mit einer oder mehreren der folgenden Funktionen: Emulgatoren, Netzmittel, Schaummittel, Gleitmittel, Antistatikum, Erhöher der Resistenz gegen Hautfette.

Dabei werden die erfindungsgemäßen Fluortenside für die Anwendung üblicherweise in entsprechend ausgelegten Zubereitungen eingebracht. Übliche Einsatzkonzentrationen sind 0.01 - 1.0 Gew.-% der erfindungsgemäßen Tenside bezogen auf die Gesamtzubereitung. Entsprechende Mittel, enthaltend die erfindungsgemäßen Fluortensiden, sind ebenfalls Gegenstand der vorliegenden Erfindung. Bevorzugt enthalten solche Mittel einen für den jeweiligen Verwendungszweck geeigneten Träger, sowie gegebenenfalls weitere Aktivstoffe und/oder gegebenenfalls Hilfsstoffe. Bevorzugte Mittel sind Farb- und Lackzubereitungen, Feuerlöschmittel, Schmierstoffe, Wasch- und Reinigungsmittel und Enteiser oder Entwicklerlösungen, Spüllösungen, Immersionsöle und Photoresists für photolithograqphische Verfahren, insbesondere für Immersionsphotolithographie-Verfahren und insbesondere zur Herstellung gedruckter Schaltungen, Agrochemikalien, Bodenpolituren, kosmetische Produkte, kosmetische Produkte oder Hydrophobiermittel zur Textilausrüstung oder Glasbehandlung. Bei bevorzugten Mitteln handelt es sich dabei um Farb- und Lackzubereitungen und Druckfarben.

Außerdem sind auch wasserbasierte Lackformulierungen, die die erfindungsgemäßen Fluortenside allein oder im Gemische mit Additiven enthalten, Gegenstand der vorliegenden Erfindung. Bevorzugt werden Lackformulierungen auf Basis der folgenden synthetischen Filmbildner verwendet: Polykondensationsharze wie Alkydharze, gesättigt/ungesättigte Polyester, Polyamide/imide, Silikonharze; Phenolharze; Harnstoffharze und Melaminharze, Polyadditionsharze wie Polyurethane und Epoxidharze, Polymerisationsharze wie Polyolefine, Polyvinylverbindungen und Polyacrylate.

Außerdem sind die erfindungsgemäßen Fluortenside auch zum Einsatz in Lacken auf Basis von Naturstoffen und modifizierten Naturstoffen geeignet. Bevorzugt sind Lacke auf Basis von Ölen, Polysacchariden wie Stärke und Cellulose als auch auf Basis von Naturharzen wie cyclischen Oligoterpenen, Polyterpenen und/oder Schellack.

Die erfindungsgemäßen Fluortenside können sowohl in physikalisch härtenden (Thermoplaste) als auch in vernetzenden (Elastomere und Duromere) wässrigen Lacksystemen verwendet werden. Bevorzugt verbessern die erfindungsgemäßen Fluortenside die Verlaufs- und Benetzungseigenschaften der Lacksysteme.

Alle hier genannten Verwendungen erfindungsgemäß einzusetzender Fluortenside, insbesondere der bevorzugten Verbindungen, sind Gegenstand der vorliegenden Erfindung. Die jeweilige Anwendung von Fluortenside zu den genannten Zwecken ist dem Fachmann bekannt, so dass der Einsatz der erfindungsgemäß einzusetzenden Fluortenside keine Probleme bereitet.

Die vollständigen Offenbarungen aller aufgeführten Anmeldungen und Veröffentlichungen sind durch Bezugnahme in diese Anmeldung eingeführt. Für die vorliegende Erfindung bedeutet sowohl die Pluralform eines Begriffs als auch die Singularform eines Begriffs auch die jeweils andere Form, soweit nicht ausdrücklich anderes angegeben ist. Alle Merkmale der vorliegenden Erfindung können in jeder Art miteinander kombiniert werden, es sei denn dass sich bestimmte Merkmale ausschließen. Dies gilt vor allem für bevorzugte und besonders bevorzugte Merkmale. Weitere Merkmale, Vorteile und Varianten der Erfindung ergeben sich auch aus den Ansprüchen und Beispielen. Die folgenden Beispiele erläutern die vorliegende Erfindung näher, ohne den Schutzbereich zu beschränken.

## Beispiele

Die NMR-Spektren werden mit einem Bruker 400 MHz Spektrometer mit internem Standard gemessen.

Die IR-Spektren werden mit einem Brucker Alpha Platinum-ATR Spektrometer gemessen.

### Bestimmung der statischen Oberflächenspannung

Es werden die statischen Oberflächenspannungen $\gamma$ von wässrigen Tensidlösungen mit verschiedenen Konzentrationen c (Gramm pro Liter) bestimmt.

Gerät: Tensiometer der Firma Dataphysics (Modell DCAT 11) Temperatur der Messlösungen: 20°±0,2°C
Eingesetzte Messmethode: Messung der Oberflächenspannung mit der Wilhelmy Plattenmethode nach DIN EN 14370.
Platte: Platin, Länge= 19,9mm

Bei der Plattenmethode wird die Oberflächen- bzw. Grenzflächenspannung der Tensidlösung aus der auf die

benetzte Länge einer Platte wirkenden Kraft nach folgender Formel berechnet.

$$\gamma = \frac{F}{L \cdot \cos\theta} = \frac{F}{L}$$

$\gamma$= Grenz- oder Oberflächenspannung; F= auf die Waage wirkende Kraft; L= benetzte Länge (19,9 mm); $\theta$= Kontaktwinkel)Die Platte besteht aus angerautem Platin und wird also optimal benetzt, so dass der Kontaktwinkel $\theta$ nahe bei 0° liegt. Der Term cos $\theta$ erreicht daher annähernd den Wert 1, so dass nur noch die gemessene Kraft und die Länge der Platte berücksichtigt werden müssen.

**Abkürzungen**

| | |
|---|---|
| EO | Ethylenoxideinheiten |
| THF | Tetrahydrofuran |
| MTBE | tert-Butylmethylether |
| Sdp. | Siedepunkt |
| w% | Gewichtsprozent |

**Beispiel 1:** Generelle Arbeitsweise Ethoxylierung

Für die Ethoxylierung wird der entsprechende Alkohol nach Stand der Technik mit einem Katalysator (z.B. Kaliumhydroxid) unter Inertgas in einem Druckreaktor geben und eine entsprechende Menge Ethylenoxid einkondensiert (Sdp: 10,5°C). Der Reaktor wird verschlossen und bei ca. 5 bar auf 80-150°C aufgeheizt. Nach Beendigung der Reaktion wird das Gemisch entspannt und eventuelle Nebenprodukte unter vermindertem Druck aus dem Produkt entfernt.

**Beispiel 2:**

An das Isopropylidenglycerol (Sigma-Aldrich) werden wie unter Beispiel 1 beschrieben 6 EO angelagert.

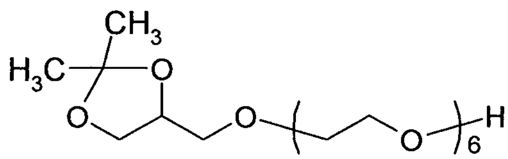

NMR-Analytik:

$^{1}$H-NMR (DMSO): 4.13 CH (m), 3.93 CH (dd), 3.7-3.3 -14 $CH_2$ (m), 1.26 $CH_3$ (s), 1.21 $CH_3$ (s)

$^{13}$C-NMR (DMSO):108. 8 **quart. C;** 74.7, 72.8, 72.0, 70.6, 70.2, 66.5, 60.7 **sec. C;** 27.0, 25.8 **prim. C**

25 g dieser Verbindung werden in 125 ml THF gelöst und in einem Dreihalskolben mit Rückflusskühler, Thermometer, Rührvorrichtung und Septum vorgelegt. Unter Schutzgasatmosphäre werden 9 g Kalium-tert.-butylat (Sigma-Aldrich) zugegeben, wobei ein leichter Temperaturanstieg (auf 32°C) beobachtet wird. Nach 20 min wird langsam 10 g Dimethylsulfat (Sigma-Aldrich) zugegeben. Es ist drauf zu achten, dass bei der Zugabe die Temperatur 37 °C nicht übersteigt. Nach einer Stunde Rühren bei Raumtemperatur wird das Reaktionsgemisch für 2 Stunden bis zur Siedetemperatur erhitzt.

Nach Abkühlung auf Raumtemperatur wir der Ansatz mit 50 ml 10%iger $NH_3$-Lösung versetzt und 1 Stunde nachgerührt um restliches Dimethylsulfat zu zersetzen.

Der Ansatz wird mit 20 mL 2N HCl versetzt (pH=1) und 6 Stunden unter Rückfluss erwärmt um die Isopropylidenschutzgruppe zu entfernen.

Das Lösemittel wird vollständig entfernt und der Rückstand in 50 ml Aceton dispergiert.

Salzrückstände werden über eine Fritte abgetrennt und das Lösemittel unter reduziertem Druck abdestilliert.

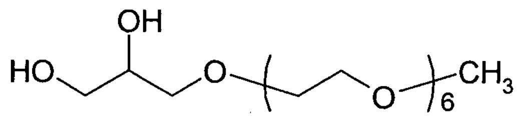

NMR-Analytik:

$^1$H-NMR (DMSO): 3.8-3.3 -***14*** $CH_2$ (m), 3.2 $OCH_3$ (s)

5 g dieser Verbindung werden in einem Autoklaven mit 11 g 1,1,1,2,2,3,3 Heptafluoro-3-trifluorovinyloxypropan (ABCR) versetzt, 10 ml Acetonitril und 2,4 g Kaliumcarbonat vermischt und für 66 Stunden auf 80°C erwärmt (Innendruck 3,7 bar). Das Rektionsgemisch wird mit 100 ml Wasser und 100 ml MTBE versetzt die Phasen getrennt und die wässrige Phase mit 2 x 50 ml MTBE ausgeschüttelt. Die vereinigten organischen Phasen werden erst mit 50 ml Wasser und dann mit 50 ml gesättigter NaCl Lösung gewaschen, über $Na_2SO_4$ getrocknet und das Lösungsmittel unter vermindertem Druck abgetrennt.

Die Ausbeute beträgt 6 g einer viskosen, bernsteinfarbenen Substanz. Folgende Struktur ergibt sich aus den ermittelten spektroskopischen Daten (NMR):

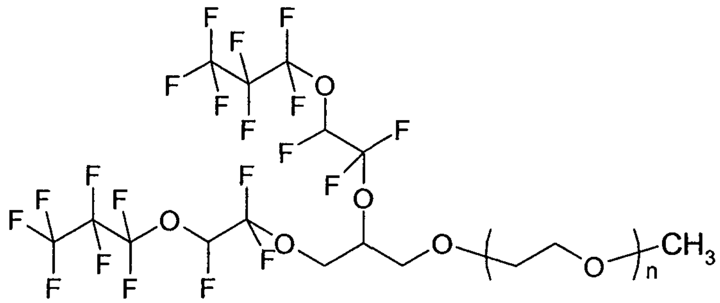

n~6

NMR-Analytik

$^1$H-NMR (DMSO): 5.9 CHF (d), 3.8-3.4 ~***14*** $CH_2$ (m), 3.3 $OCH_3$ (s)

Statische Oberflächenspannung:

$\gamma$ 18,9 mN/m (0,1 w%); CMC [g/l] 0,01

**Beispiel 3:**

An das Isopropylidenglycerol werden wie unter Beispiel 1 beschrieben 12 EO angelagert.

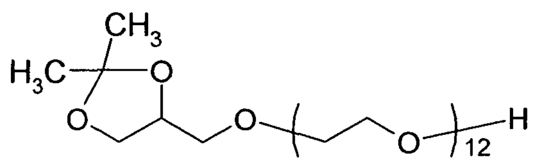

NMR-Analytik

$^1$H-NMR (DMSO): 4.13 CH (1 m), 3.93 C**H** (1 dd), 3.7-3.3 C$\mathbf{H_2}$ (26, m), 1.26 C$\mathbf{H_3}$ (3, s), 1.21 C$\mathbf{H_3}$ (3, s)

Die weitere Umsetzung erfolgt analog Beispiel 2

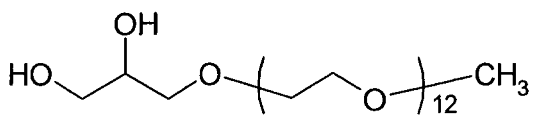

NMR-Analytik:

$^1$H-NMR (DMSO): 3.8-3.3 C**H**$_2$ (26, m), 3.2 OC**H**$_3$ (3, s)

Die Ausbeute beträgt 7,6 g einer viskosen, bernsteinfarbenen Substanz. Folgende Struktur ergibt sich aus den ermittelten spektroskopischen Daten (NMR):

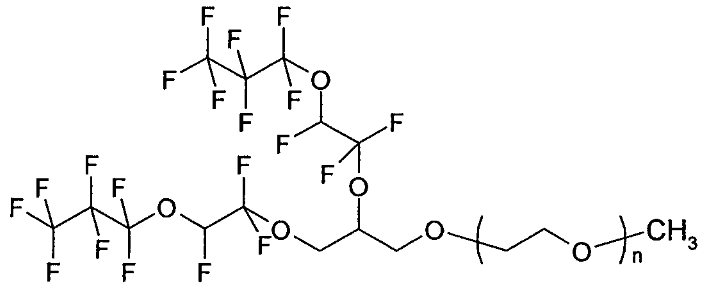

n~12

NMR-Analytik:

$^1$H-NMR (DMSO): 5.9 C**H**F (2, d), 3.8-3.4 CH (-26, m), 3.3 OC**H**$_3$ (3, s)

Statische Oberflächenspannung:

$\gamma$ 18,0 mN/m (0,1 w%)

**Beispiel 4:**

3700 g 1,1,2,2,3,3 Hexafluoro-1-trifluoromethoxy-3-trifluorovinyloxypropan werden in einem Autoklaven mit 500 g Glycerin-1-acetat, 670 g Kaliumcarbonat und 2300 g Acetonitril und für 68 Stunden auf 80°C erwärmt (Innendruck 2,3 bar).

Der Ansatz wird mit Wasser gewaschen und die organische Phase abgetrennt. Der Ansatz wird mit 500mL 32%-ige Natronlauge und 500mL Wasser versetzt und 72 Stunden bei Siedetemperatur gerührt.

Das Produkt wird mit MTBE extrahiert, erneut mit Wasser gewaschen, über $Na_2SO_4$ getrocknet und im Vakuum fraktioniert destilliert Hauptfraktion (Sdp 82 °C bei 0,37 mbar).

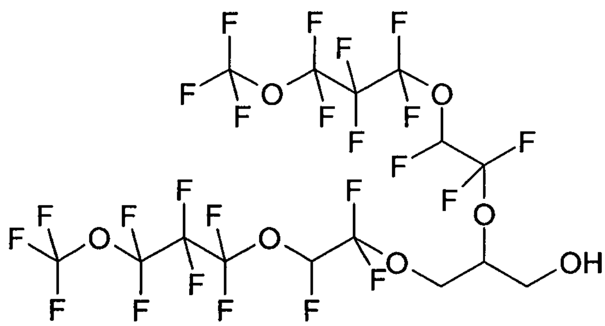

NMR-Analytik

$^1$H-NMR (DMSO): 6.6 C**H**F (2, m), 5.2 - 3.7 C**H** (5, m)

Der teilfluorierte Alkohol wird analog Beispiel 1 ethoxyliert wobei 12 EO angelagert werden. Folgende Struktur ergibt sich aus den ermittelten spektroskopischen Daten (NMR:

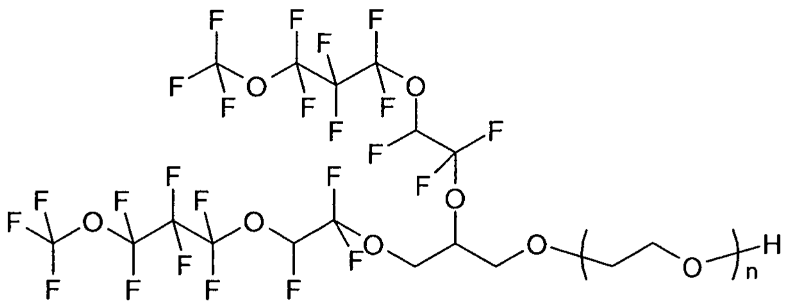

n~12

NMR-Analytik

$^1$H-NMR (DMSO): 6.8 C**H**F (2, d), 4.9-4.0 C**H** (5, m), 3.8-3.2 $CH_2C\mathbf{H}_2O$ (48, m)

Statische Oberflächenspannung:

$\gamma$ 18,62 mN/m (0,1w%)

**Beispiel 5:**

Analog Beispiel 5 wird ein Tenside mit 24 EO Einheiten synthetisiert. Folgende Struktur ergibt sich aus den ermittelten spektroskopischen Daten (NMR):

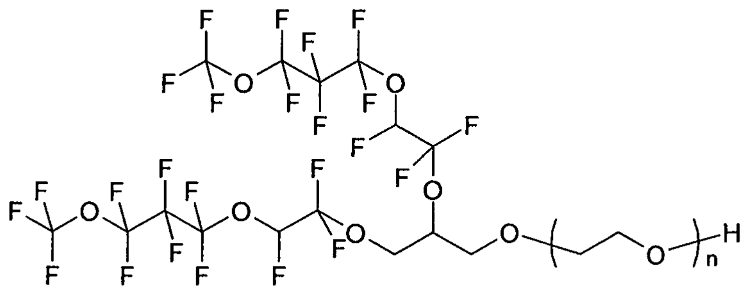

n~24

NMR-Analytik

$^1$H-NMR (DMSO): 6.8 C**H**F (2, d), 4.9-4.0 C**H** (5, m), 3.8-3.2 $CH_2C\mathbf{H}_2O$ (-96, m)

Statische Oberflächenspannung:

$\gamma$ 17,58 mN/m (0,1 w%)

**Beispiel 6:**

3,12g (0,056mol) Kaliumhydroxid werden mit 15ml Acetonitril und 2,83g (0,024mol) 3-Dimethylamino-propan-1,2-diol (ABCR) im Druckreaktor zusammen gegeben. Anschließend werden 13,90g (0,052mol) 1,1,1,2,2,3,3-Heptafluor-3-trifluorvinyloxypropan dazu gegeben. Der Druckreaktor wird verschlossen und das Gemisch auf 80°C erhitzt. Es wird 18 Stunden bei dieser Reaktion nachgerührt.

Das Reaktionsgemisch wird nach Beendigung der Reaktion abkühlen gelassen, filtriert und das Lösemittel im Vakuum entfernt.

Zur weiteren Aufarbeitung wird das Produkt unter Vakuum fraktioniert destilliert.

Man erhält 9,66g (63%) hellgelbes Öl.

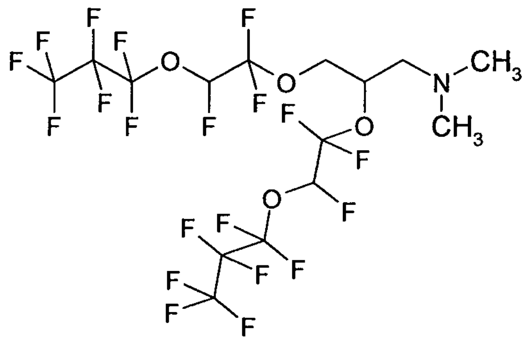

NMR-Analytik:

$^1$H-NMR (DMSO): 6.89-6.65 C**H**F (2, dt), 5.20 C**H** (1, tt), 4.34-4.16 C**H**$_2$ (2m), 3.56-3.37 C**H**$_2$ (2m), 2.88 C**H**$_3$ (6, s)

7,4g (0,011mol) [2,3-Bis-(1,1,2-trifluor-2-heptafluorpropyloxyethoxy)-propyl]-dimethylamin, 1,41g (0,012mol) Chloressigsäure Natriumsalz (VWR) und 10mL Ethanol 90% werden in einen Rundkolben gegeben und 96 h am Rückfluss gerührt.

Das Reaktionsgemisch wird filtriert und das Lösemittel im Vakuum entfernt. Das Rohprodukt wird auf Kieselgel gegeben und mit Toluol/Essigester 2/1 vom Edukt getrennt.

Das Produkt wird mit Aceton vom Kieselgel gewaschen. Ausbeute 4,4 g.

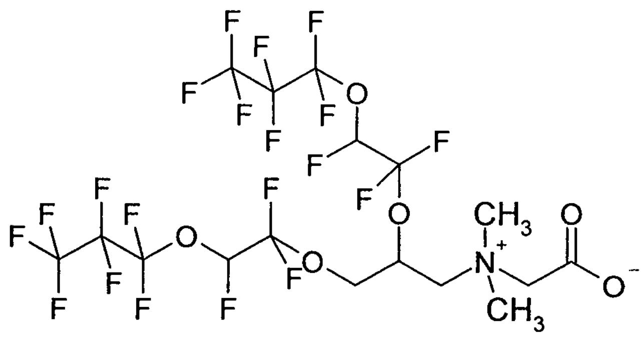

NMR-Analytik:

$^1$H-NMR (DMSO): 6.89-6.65 C**H**F (2, dd), 5.5 C**H** (1, s), 4.54-4.16 C**H**$_2$ (4m), 4.0 C**H**$_2$ (2,s), 3.40 C**H**$_3$ (6, ds)

Statische Oberflächenspannung:

15,89 mN/m (1.0 w%)

**Beispiel 7:**

5,0g (0,034mol) 3-Hydroxy-2-hydroxymethyl-2-methyl-propionsäuremethyl ester (VWR), 15mL (0,084mol) 1,1,1,2,2,3,3-Heptafluor-3-trifluorvinyloxypropan, 6,1g (0,044mol) Kaliumcarbonat und 25mL Acetonitril werden im Druckreaktor zusammen gegeben und auf 80°C erhitzt. Es wird für 48 Stunden bei dieser Temperatur nachgerührt.

Anschließend wird abkühlen gelassen, das Gemisch filtriert und das Lösemittel wird im Vakuum entfernt.

Das Rohprodukt wird über KG gefrittet und man erhält 17,1g (0,025mol) 2-Methyl-3-(1,1,2-trifluor-2-heptafluorpropyloxy-ethoxy)-2-(1,1,2-trifluor-2-heptafluorpropyloxy-ethoxymethyl)-propionsäuremethylester.

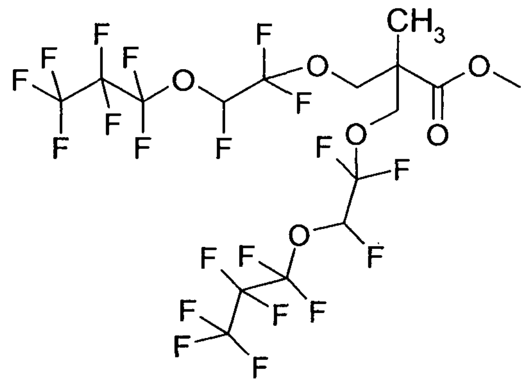

10,00g (0,015mol) 2-Methyl-3-(1,1,2-trifluor-2-heptafluorpropyloxy-ethoxy)-2-(1,1,2-trifluor-2-heptafluorpropyloxy-ethoxymethyl)-propionsäure methyl ester werden mit 1,47 g (0,037mol) NaOH in 15ml (0,257mol) Ethanol zusammen geben und für 18 Stunden am Rückfluss gerührt. Anschließend wird das Lösemittel im Vakuum entfernt, der Rückstand in Aceton suspendiert und filtriert das Natriumhydroxid zu entfernen.

Das Aceton wird im Vakuum abdestilliert und man erhält 9,4g (0,014mol) des entsprechenden Fluortensid als Natriumsalz.

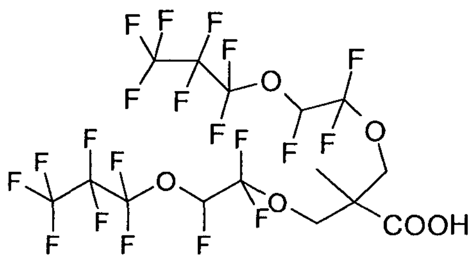

Charakterisierung per IR: $COO^-$ st as $1607cm^{-1}$

Statische Oberflächenspannung:

16,45 mN/m (1.0 w%)

## Patentansprüche

1. Verbindungen der Formeln (I), (IIa) oder (IIb)

$Z_nSpacerX_x$ (I)

wobei

$Z = R_f\text{-}CHF\text{-}CF_2\text{-}Y\text{-}(CH_2)_m\text{-}$ oder
$R_f\text{-}O\text{-}CHF\text{-}CF_2\text{-}Y\text{-}(CH_2)_m\text{-}$ oder
$R_f\text{-}O\text{-}(CF_2)_{1\text{-}4}\text{-}CHF\text{-}CF_2\text{-}Y\text{-}(CH_2)_m\text{-}$ oder
$R_f\text{-}O\text{-}(CF_2)_{1\text{-}4}\text{-}O\text{-}CHF\text{-}CF_2\text{-}Y\text{-}(CH_2)_m\text{-}$ oder
$R_f\text{-}O\text{-}(CF(CF_3)\text{-}CF_2)_{1\text{-}4}\text{-}CHF\text{-}CF_2\text{-}Y\text{-}(CH_2)_m\text{-}$ oder
$R_f\text{-}O\text{-}(CF(CF_3)\text{-}CF_2)_{1\text{-}4}\text{-}O\text{-}CHF\text{-}CF_2\text{-}Y\text{-}(CH_2)_m\text{-}$ oder
$R_f\text{-}O\text{-}(CF_2\text{-}O)_{1\text{-}4}\text{-}CHF\text{-}CF_2\text{-}Y\text{-}(CH_2)_m\text{-}$ oder
$R_f\text{-}O\text{-}(CF_2\text{-}CF_2\text{-}O)_{1\text{-}4}\text{-}CHF\text{-}CF_2\text{-}Y\text{-}(CH_2)_m\text{-}$ oder
$R_f\text{-}O\text{-}(CF_2\text{-}O)_{1\text{-}4}\text{-}(CF_2\text{-}CF_2\text{-}O)_{1\text{-}4}\text{-}CHF\text{-}CF_2\text{-}Y\text{-}(CH_2)_m$
n = 2 oder 3,
$R_f = CF_3\text{-}, CF_3\text{-}CF_2\text{-}$ oder $CF_3\text{-}CF_2\text{-}CF_2\text{-}$,
Y = O oder S,

m = 0 oder 1,
Spacer = eine gesättigte, verzweigte oder unverzweigte Kohlenwasserstoffeinheit, wobei ein oder mehrere nicht benachbarte C-Atome durch O oder N ersetzt sein können,
X eine der Gruppen $-SO_3^-$, $-OSO_3^-$, $-COO^-$, $-PO_3^{2-}$, $OPO_3^{2-}$, Polyethylen- oder Polypropylenglykole, $-CH(OH)-CH_2-NH-Sach$, $-Y-(CH_2-CH_2-O)_v-R^4$, Betaine oder Sulfobetaine ist, mit Sach = verschiedene Zucker und Y = S, O oder NH, bevorzugt O, $R^4$ = H oder Alkyl, bevorzugt H oder $CH_3$, und v = 1-100, bevorzugt 1-30 ist, x = 1,

$$[F(CF_2)_{n'}(O)_oCHF-CF_2-Y-(CH_2)_m]_2Spacer-X \qquad \text{Formel (IIa)}$$

$$[F(CF_2)_{n'}(O)_o-(CF_2)_{1-4}-O-CHF-CF_2-Y-(CH_2)_m]_2Spacer-X \qquad \text{Formel (IIb)}$$

mit n' = 1-6, m = 0 oder 1, o = 0 oder 1,
Y = O oder S, und die Gruppierung (Spacer-X) = $CR^5(CH_2)_{n''}OH$, $CR^5(CH_2)_{n''}SH$, $CR^5(CH_2)_{n''}COOH$, $CR^5(CH_2)_{n''}SO_3H$, $CR^5(CH_2)_{n''}NH_2$, $CR^5(CH_2)_{n''}NR'_2$, $CR^5(CH_2)_{n''}N^+(CH_3)_3Cl^-$,
$CR^5(CH_2)_{n''}NR'_2-CH_2-COO^-$, $CR^5(CH_2)_{n''}O(CHR^a-CHR^bO)_nR''$,
$CR^5(CH_2)_{n''}S(CHR^a-CHR^bO)_nR''$ oder $CR^5(CH_2)_{n''}NH(CHR^a-CHR^bO)_nR''$, n'' = 0 oder 1, n = 1-30, und $R^5$, R', R'', $R^a$ und $R^b$ unabhängig voneinander = H oder Alkyl
und wobei alle Indizes so gewählt sind, dass keine O-O Bindungen vorliegen.

2. Verbindungen gemäß Anspruch 1, **dadurch gekennzeichnet, dass** der Spacer eine gesättigte, verzweigte oder unverzweigte Alkylengruppe ist, wobei ein oder mehrere nicht benachbarte C-Atome durch O oder N ersetzt sein können.

3. Verbindungen gemäß einem oder mehreren der Ansprüche 1 bis 2, **dadurch gekennzeichnet, dass** Z gleich:

$R_f-CHF-CF_2-Y-(CH_2)_m-$ oder
$R_f-O-CHF-CF_2-Y-(CH_2)_m-$ oder
$R_f-O-(CF_2)_{1-4}-CHF-CF_2-Y-(CH_2)_m-$ oder
$R_f-O-(CF_2)_{1-4}-O-CHF-CF_2-Y-(CH_{2)m}-$ ist mit m = 0 oder 1.

4. Verbindungen gemäß einem oder mehreren der Ansprüche 1 bis 3, **dadurch gekennzeichnet, dass** Z gleich:
$R_f-O-CHF-CF_2-Y-(CH_2)_m-$ oder $R_f-O-(CF_2)_{1-4}-O-CHF-CF_2-Y-(CH_{2)m}-$ist mit m = 0 oder 1.

5. Verbindungen gemäß einem oder mehreren der Ansprüche 1 bis 4, **dadurch gekennzeichnet, dass** Rf = $CF_3$- oder $CF_3-CF_2-CF_2$- ist.

6. Verbindungen gemäß einem oder mehreren der Ansprüche 1 bis 5, **dadurch gekennzeichnet, dass** die Gruppierung (Spacer-X) in der Formel (I) eine der folgenden Bedeutungen hat:

$CR^5(CH_2)_{n''}OH$, $CR^5(CH_2)_{n''}SH$, $CR^5(CH_2)_{n''}COOH$, $CR^5(CH_2)_{n''}SO_3H$,
$CR^5(CH_2)_{n''}NH_2$, $CR^5(CH_2)_{n''}NR'_2$, $CR^5(CH_2)_{n''}N^+(CH_3)_3Cl^-$,
$CR^5(CH_2)_{n''}NR'_2-CH_2-COO^-$, $CR^5(CH_2)_{n''}O(CHR^a-CHR^bO)_nR''$,
$CR^5(CH_2)_{n''}S(CHR^a-CHR^bO)_nR''$ oder $CR^5(CH_2)_{n''}NH(CHR^a-CHR^bO)_nR''$, mit n'' = 0 oder 1, n = 1-30 und $R^5$, R', R'', $R^a$ und $R^b$ unabhängig voneinander = H oder Alkyl.

7. Verbindungen gemäß einem oder mehreren der Ansprüche 1 bis 6, **dadurch gekennzeichnet, dass** sie der Formel (IIa) oder (IIb) entsprechen:

$$[F(CF_2)_{n'}(O)_oCHF-CF_2-Y-(CH_2)_m]_2Spacer-X \qquad \text{Formel (IIa)}$$

$$[F(CF_2)_{n'}(O)_o-(CF_2)_{1-4}-O-CHF-CF_2-Y-(CH_2)_m]_2Spacer-X \qquad \text{Formel (IIb)}$$

mit n' = 1-3, m = 0 oder 1, o= 0 oder 1,
Y = O, und die Gruppierung (Spacer-X) = $CR^5(CH_2)_{n''}OH$, $CR^5(CH_2)_{n''}SH$, $CR^5(CH_2)_{n''}COOH$, $CR^5(CH_2)_{n''}SO_3H$,

$CR^5(CH_2)_{n''}NH_2$, $CR^5(CH_2)_{n''}NR'_2$, $CR^5(CH_2)_{n''}N^+(CH_3)_3Cl^-$, $CR^5(CH_2)_{n''}NR'_2-CH_2-COO^-$, $CR^5(CH_2)_{n''}O(CHR^a-CHR^bO)_nR''$, $CR^5(CH_2)_{n''}S(CHR^a-CHR^bO)_nR''$ oder $CR^5(CH_2)_{n''}NH(CHR^a-CHR^bO)_nR''$, n" = 0 oder 1, n = 1-30, und $R^5$, R', R", $R^a$ und $R^b$ unabhängig voneinander = H oder Alkyl.

**8.** Verbindungen gemäß einem oder mehreren der Ansprüche 1 bis 7, **dadurch gekennzeichnet, dass** n' = 1-3, m = 0 oder 1, o = 1, Y = O, und R', R", $R^a$ und $R^b$ unabhängig voneinander = H oder C1-C4 Alkyl.

**9.** Verbindungen gemäß einem oder mehreren der Ansprüche 1 bis 8, **dadurch gekennzeichnet, dass** sie der Formel (III), (III'), (IV) oder (IV') entsprechen:

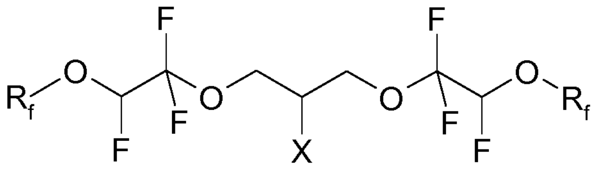

Formel (III)

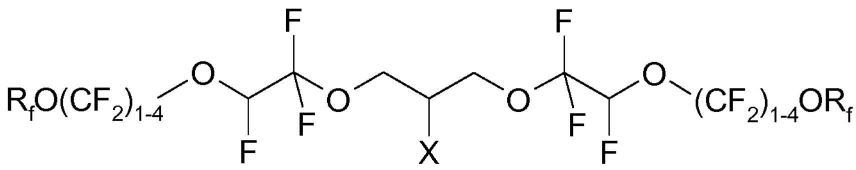

Formel (III')

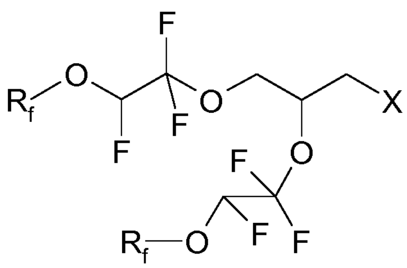

Formel (IV)

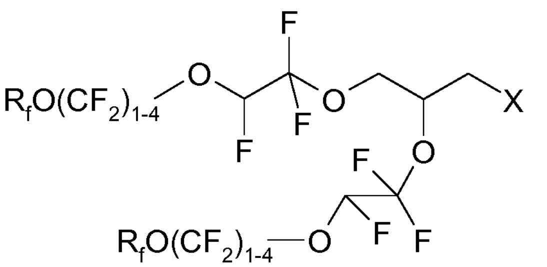

Formel (IV')

mit $R_f$ = fluoriertes Alkyl, linear oder verzweigt, bevorzugt perfluoriertes C1-C4-Alkyl, und X eine hydrophile Gruppe ist.

**10.** Verbindungen gemäß einem oder mehreren der Ansprüche 1 bis 9, **dadurch gekennzeichnet, dass** sie den Formeln (IIIa), (III'a), (IIIb), (III'b), (IVa), (IV'a), (IVb) (IV'ba), (IVc) oder (IV'c) entsprechen:

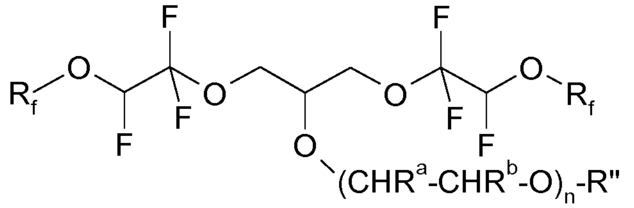
Formel (IIIa)

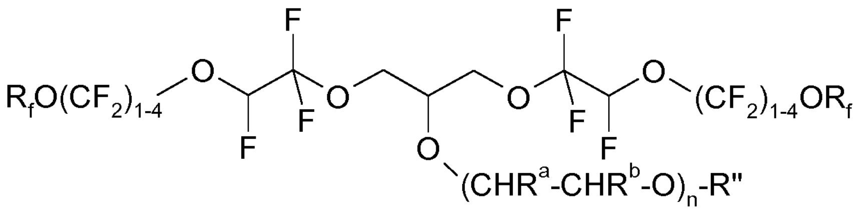
Formel (III'a)

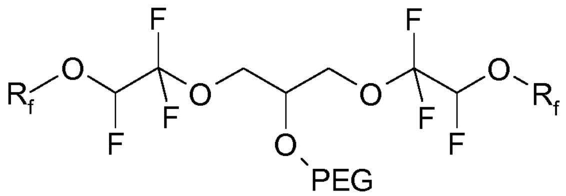
Formel (IIIb)

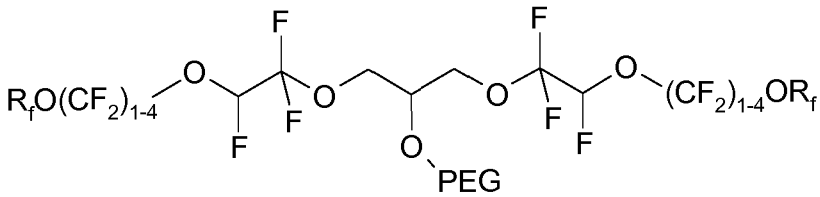
Formel (III'b)

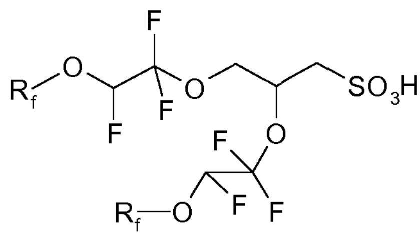
Formel (IVa)

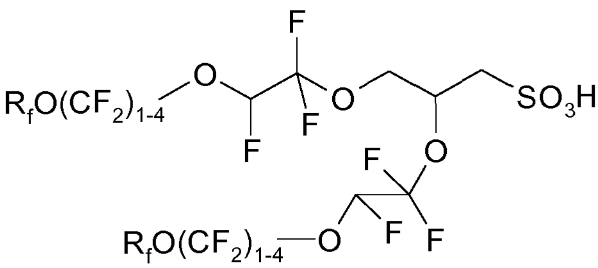
Formel (IV'a)

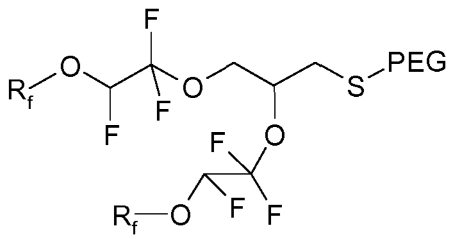

Formel (IVb)

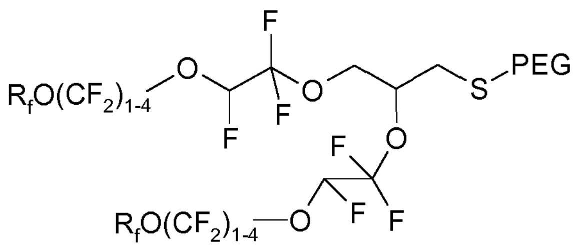

Formel (IV'b)

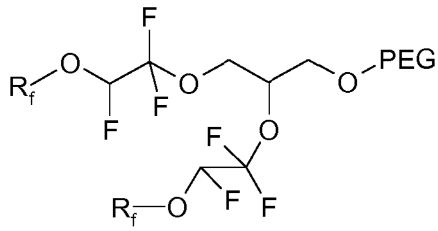

Formel (IVc)

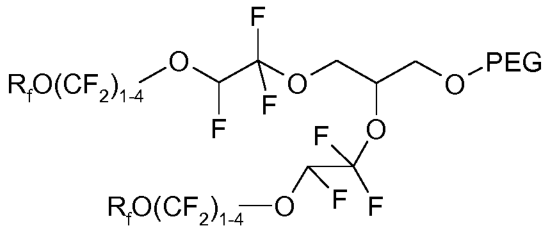

Formel (IV'c)

wobei $R_f$ = fluoriertes Alkyl, linear oder verzweigt, bevorzugt perfluoriertes C1-C4-Alkyl, und PEG für Polyethylenglycol, Polypropylenglycol, Polyethylenglycolalkylether oder Polypropylenglycolalkylether steht.

**11.** Verbindungen gemäß einem oder mehreren der Ansprüche 1 bis 10, **dadurch gekennzeichnet, dass** sie einer der Formeln (V) bis (XII) entsprechen:

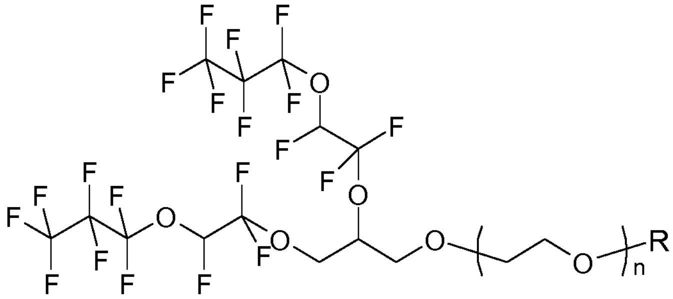

n~6 und R=H oder Methyl

Formel (V)

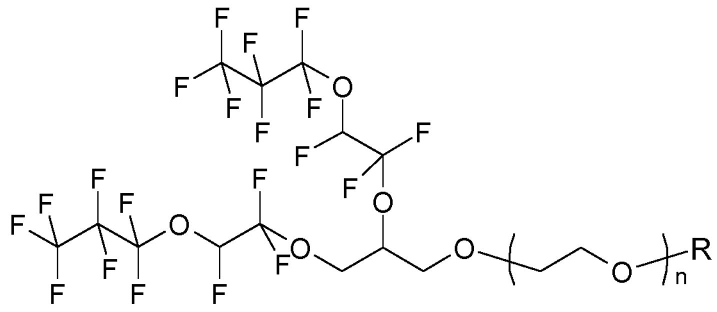

n~12 und R=H oder Methyl Formel (VI)

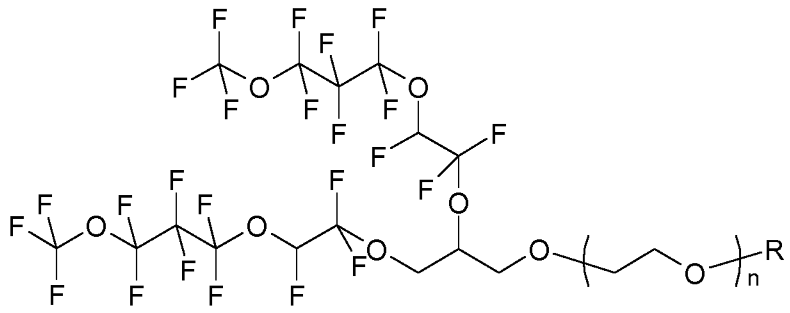

n~12 und R=H oder Methyl Formel (VII)

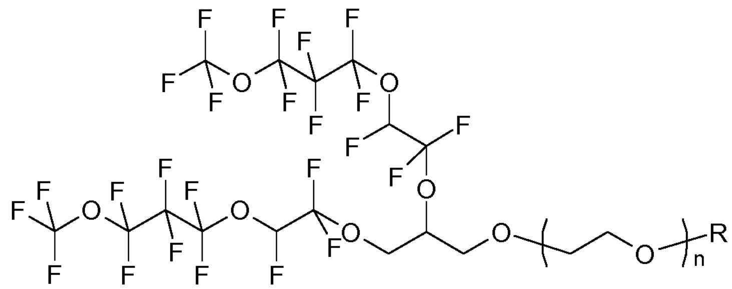

n~24 und R=H oder Methyl Formel (VIII)

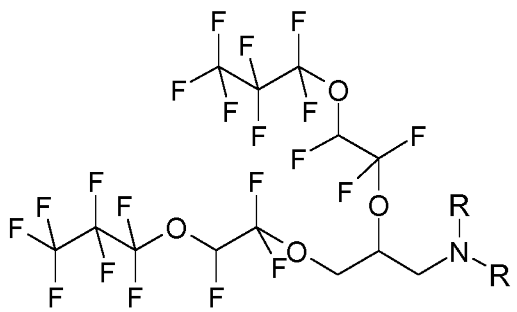

R=Methyl oder Ethyl Formel (IX)

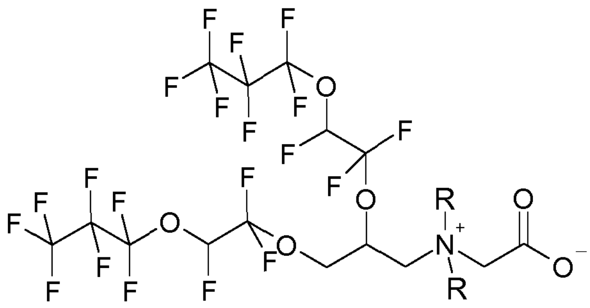

R=Methyl oder Ethyl Formel (X)

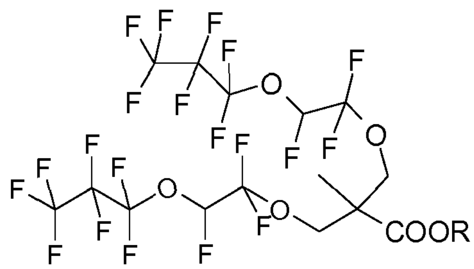

R=H, Methyl oder Ethyl Formel (XI)

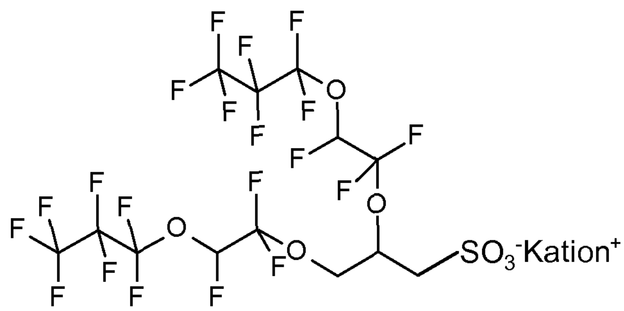

Kation$^{+}$ = $Na^{+}$, $K^{+}$ oder $NH_4^{+}$ Formel (XII)

**12.** Verwendung von Verbindungen gemäß einem oder mehreren der Ansprüche 1 bis 11 als Additive in Farben, Lacken, Druckfarben, Schutzanstrichen, Spezialcoatings in elektronischen oder optischen Anwendungen, Photolacken, Top Antireflective Coatings oder Bottom Antireflective Coatings, Entwicklerlösungen und Waschlösungen und Photoresists für photolithograqphische Verfahren, kosmetischen Produkten, Agrochemikalien, Bodenpolituren, photographischen Beschichtungen oder Beschichtungen optischer Elemente.

**13.** Mittel enthaltend eine Verbindung gemäß einem oder mehreren der Ansprüche 1 bis 11 und einen für den jeweiligen Verwendungszweck geeigneten Träger sowie ggf. weitere spezifische Aktivstoffe.

**14.** Mittel nach Anspruch 13, **dadurch gekennzeichnet, dass** es sich bei dem Mittel um Farb- und Lackzubereitungen, Feuerlöschmittel, Schmierstoffe, Wasch- und Reinigungsmittel, Enteiser, Entwicklerlösungen und Waschlösungen und Photoresists für photolithograqphische Verfahren, kosmetische Produkte, Agrochemikalien, Bodenpolituren oder Hydrophobiermittel zur Textilausrüstung oder Glasbehandlung handelt.

## Claims

1. Compounds of the formulae (I), (IIa) or (IIb)

$$Z_nSpacerX_x \qquad (I)$$

where

Z = $R_f$-CHF-$CF_2$-Y-$(CH_2)_m$- or
$R_f$-O-CHF-$CF_2$-Y-$(CH_2)_m$- or
$R_f$-O-$(CF_2)_{1-4}$-CHF-$CF_2$-Y-$(CH_2)_m$- or
$R_f$-O-$(CF_2)_{1-4}$-O-CHF-$CF_2$-Y-$(CH_2)_m$- or
$R_f$-O-$(CF(CF_3)$-$CF_2)_{1-4}$-CHF-$CF_2$-Y-$(CH_2)_m$- or
$R_f$-O-$(CF(CF_3)$-$CF_2)_{1-4}$-O-CHF-$CF_2$-Y-$(CH_2)_m$- or
$R_f$-O-$(CF_2$-$O)_{1-4}$-CHF-$CF_2$-Y-$(CH_2)_m$- or
$R_f$-O-$(CF_2$-$CF_2$-$O)_{1-4}$-CHF-$CF_2$-Y-$(CH_2)_m$- or
$R_f$-O-$(CF_2$-$O)_{1-4}$-$(CF_2$-$CF_2$-$O)_{1-4}$-CHF-$CF_2$-Y-$(CH_2)_m$-,
n = 2 or 3,
Rf = $CF_3$-, $CF_3$-$CF_2$- or $CF_3$-$CF_2$-$CF_2$-,
Y = O or S,
m = 0 or 1,
spacer = a saturated, branched or unbranched hydrocarbon unit, in which one or more non-adjacent C atoms may be replaced by O or N,
X is one of the groups -$SO_3^-$, -$OSO_3^-$, -$COO^-$, -$PO_3^{2-}$ or $OPO_3^{2-}$, polyethylene glycol or polypropylene glycol, -CH(OH)-$CH_2$-NH-sach,
-Y-$(CH_2$-$CH_2$-$O)_v$-$R^4$, betaines or sulfobetaines, where sach = various sugars and Y = S, O or NH, preferably O, $R^4$ = H or alkyl, preferably H or $CH_3$, and v = 1=100, preferably 1-30,
x = 1,

$$[F(CF_2)_{n'}(O)_oCHF\text{-}CF_2\text{-}Y\text{-}(CH_2)_m]_2Spacer\text{-}X \qquad \text{formula (IIa)}$$

$$[F(CF_2)_{n'}(O)_o\text{-}(CF_2)_{1\text{-}4}\text{-}O\text{-}CHF\text{-}CF_2\text{-}Y\text{-}(CH_2)_m]_2Spacer\text{-}X \qquad \text{formula (IIb)}$$

where n' = 1-6, m = 0 or 1, o = 0 or 1,
Y = O or S, and the group (spacer-X) = $CR^5(CH_2)_{n''}OH$, $CR^5(CH_2)_{n''}SH$, $CR^5(CH_2)_{n''}COOH$, $CR^5(CH_2)_{n''}SO_3H$, $CR^5(CH_2)_{n''}NH_2$, $CR^5(CH_2)_{n''}NR'_2$, $CR^5(CH_2)_{n''}N^+(CH_3)_3Cl^-$, $CR^5(CH_2)_{n''}NR'_2$-$CH_2$-$COO^-$, $CR^5(CH_2)_{n''}O(CHR^a$-$CHR^bO)_nR''$, $CR^5(CH_2)_{n''}S(CHR^a$-$CHR^bO)_nR''$ or $CR^5(CH_2)_{n''}NH(CHR^a$-$CHR^bO)_nR''$, n" = 0 or 1, n = 1-30, and $R^5$, R', R", $R^a$ and $R^b$ independently of one another = H or alkyl,
and where all indices are selected so that no O-O bonds are present.

2. Compounds according to Claim 1, **characterised in that** the spacer is a saturated, branched or unbranched alkylene group, in which one or more non-adjacent C atoms may be replaced by O or N.

3. Compounds according to one or more of Claims 1 to 2, **characterised in that**
Z is equal to:

$R_f$-CHF-$CF_2$-Y-$(CH_2)_m$- or
$R_f$-O-CHF-$CF_2$-Y-$(CH_2)_m$- or
$R_f$-O-$(CF_2)_{1-4}$-CHF-$CF_2$-Y-$(CH_2)_m$- or
$R_f$-O-$(CF_2)_{1-4}$-O-CHF-$CF_2$-Y-$(CH_{2)m}$-, where m = 0 or 1.

4. Compounds according to one or more of Claims 1 to 3, **characterised in that**
Z is equal to:
$R_f$-O-CHF-$CF_2$-Y-$(CH_2)_m$- or $R_f$-O-$(CF_2)_{1-4}$-O-CHF-$CF_2$-Y-$(CH_{2)m}$-, where m = 0 or 1.

5. Compounds according to one or more of Claims 1 to 4, **characterised in that**
Rf = $CF_3$- or $CF_3$-$CF_2$-$CF_2$-.

6. Compounds according to one or more of Claims 1 to 5, **characterised in that** the group (spacer-X) in the formula (I) has one of the following meanings:

$CR^5(CH_2)_{n''}OH$, $CR^5(CH_2)_{n''}SH$, $CR^5(CH_2)_{n''}COOH$, $CR^5(CH_2)_{n''}SO_3H$, $CR^5(CH_2)_{n''}NH_2$, $CR^5(CH_2)_{n''}NR'_2$, $CR^5(CH_2)_{n''}N^+(CH_3)_3Cl^-$, $CR^5(CH_2)_{n''}NR'_2\text{-}CH_2\text{-}COO^-$, $CR^5(CH_2)_{n''}O(CHR^a\text{-}CHR^bO)_nR''$, $CR^5(CH_2)_{n''}S(CHR^a\text{-}CHR^bO)_nR''$ or $CR^5(CH_2)_{n''}NH(CHR^a\text{-}CHR^bO)_nR''$,
where n" = 0 or 1, n = 1-30 and $R^5$, R', R", $R^a$ and $R^b$ independently of one another = H or alkyl.

7. Compounds according to one or more of Claims 1 to 6, **characterised in that** they conform to the formula (IIa) or (IIb):

$[F(CF_2)_{n'}(O)_oCHF\text{-}CF_2\text{-}Y\text{-}(CH_2)_m]_2$spacer-X formula (IIa)

$[F(CF_2)_{n'}(O)_o\text{-}(CF_2)_{1\text{-}4}\text{-}O\text{-}CHF\text{-}CF_2\text{-}Y\text{-}(CH_2)_m]_2$spacer-X formula (IIb)

where n' = 1-6, m = 0 or 1, o = 0 or 1,
Y = O, and the group (spacer-X) = $CR^5(CH_2)_{n''}OH$, $CR^5(CH_2)_{n''}SH$, $CR^5(CH_2)_{n''}COOH$, $CR^5(CH_2)_{n''}SO_3H$, $CR^5(CH_2)_{n''}NH_2$, $CR^5(CH_2)_{n''}NR'_2$, $CR^5(CH_2)_{n''}N^+(CH_3)_3$ $Cl^-$, $CR^5(CH_2)_{n''}NR'_2\text{-}CH_2\text{-}COO^-$, $CR^5(CH_2)_{n''}O(CHR^a\text{-}CHR^bO)_nR''$, $CR^5(CH_2)_{n''}S(CHR^a\text{-}CHR^bO)_nR''$ or $CR^5(CH_2)_{n''}NH(CHR^a\text{-}CHR^bO)_nR''$, n" = 0 or 1,
n = 1-30, and $R^5$, R', R", $R^a$ and $R^b$ independently of one another = H or alkyl.

8. Compounds according to one or more of Claims 1 to 7, **characterised in that** n' = 1-3, m = 0 or 1, o = 1, Y = O, and R', R", $R^a$ and $R^b$ independently of one another = H or C1-C4 alkyl.

9. Compounds according to one or more of Claims 1 to 8, **characterised in that** they conform to the formula (III), (III'), (IV) or (IV'):

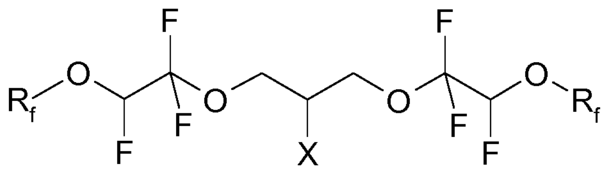

formula (III)

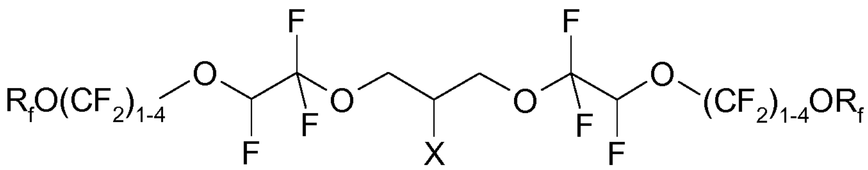

formula (III')

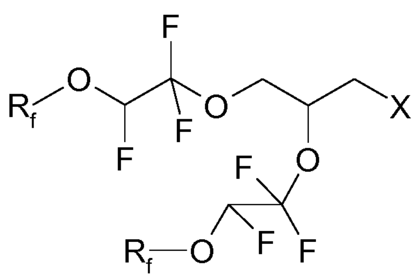

formula (IV)

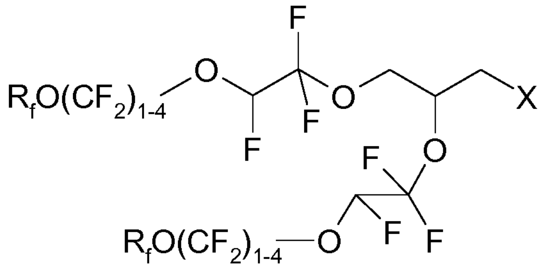

formula (IV')

where $R_f$ = fluorinated alkyl, linear or branched, preferably perfluorinated C1-C4-alkyl, and X is a hydrophilic group.

**10.** Compounds according to one or more of Claims 1 to 9, **characterised in that** they conform to the formulae (IIIa), (III'a), (IIIb), (III'b), (IVa), (IV'a), (IVb) (IV'ba), (IVc) or (IV'c):

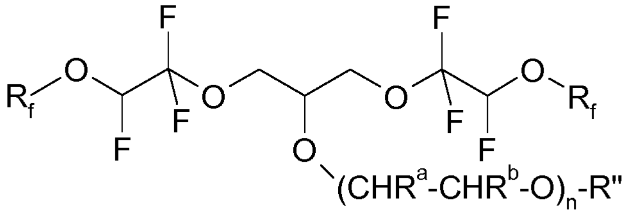

formula (IIIa)

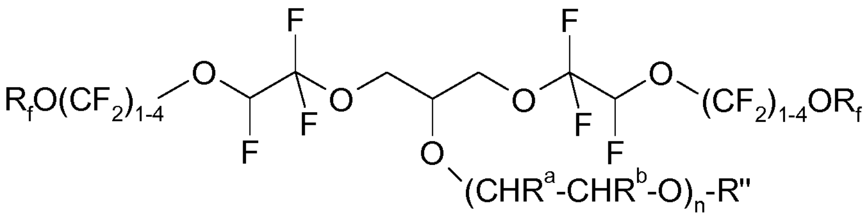

formula (III'a)

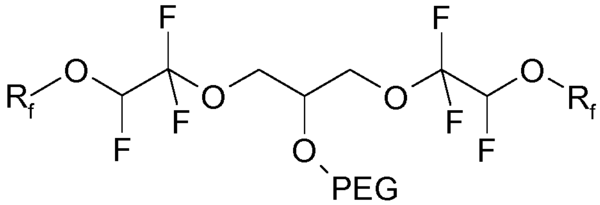

formula (IIIb)

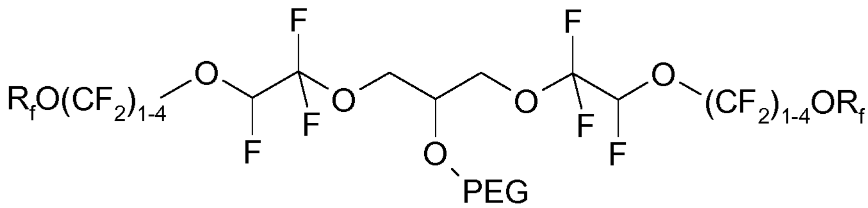

formula (III'b)

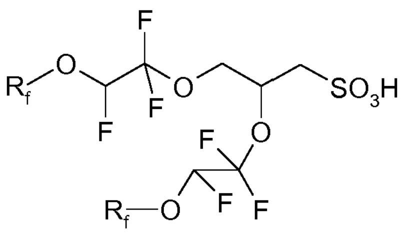

formula (IVa)

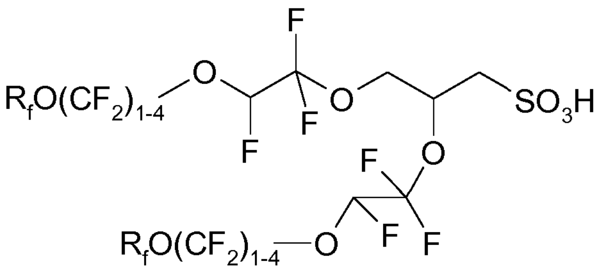

formula (IV'a)

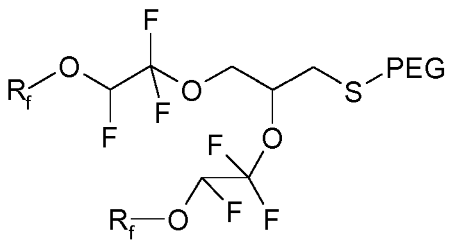

formula (IVb)

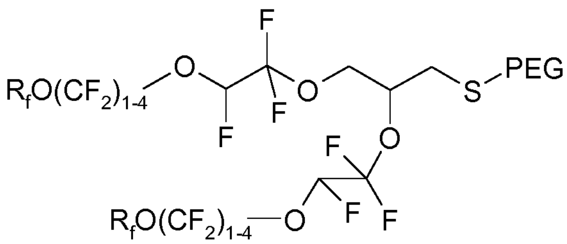

formula (IV'b)

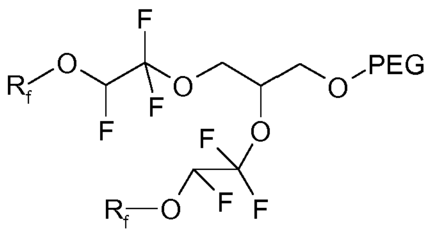

formula (IVc)

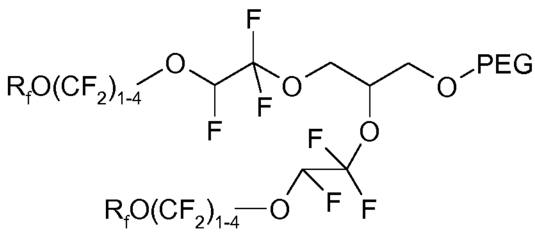

formula (IV'c)

where $R_f$ = fluorinated alkyl, linear or branched, preferably perfluorinated C1-C4-alkyl, and PEG stands for polyethylene glycol, polypropylene glycol, polyethylene glycol alkyl ether or polypropylene glycol alkyl ether.

**11.** Compounds according to one or more of Claims 1 to 10, **characterised in that** they conform to one of the formulae (V) to (XII):

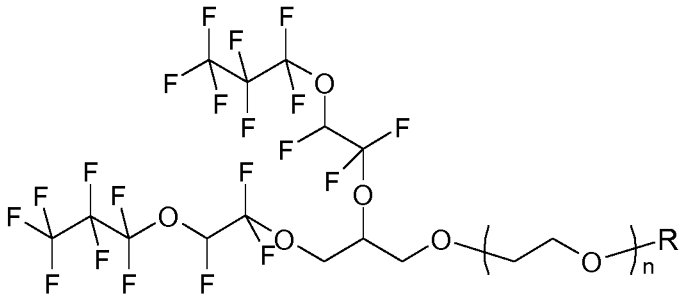

n~6 and R=H or methyl formula (V)

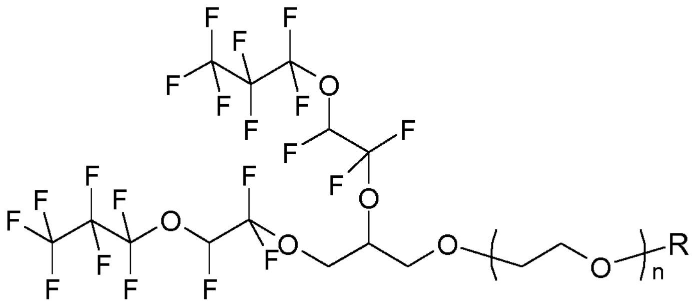

n~12 and R=H or methyl formula (VI)

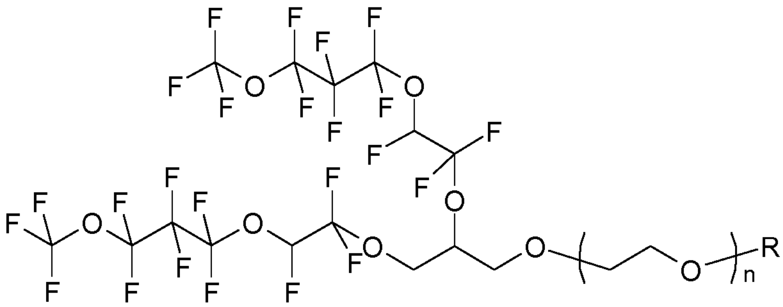

n~12 and R=H or methyl formula (VII)

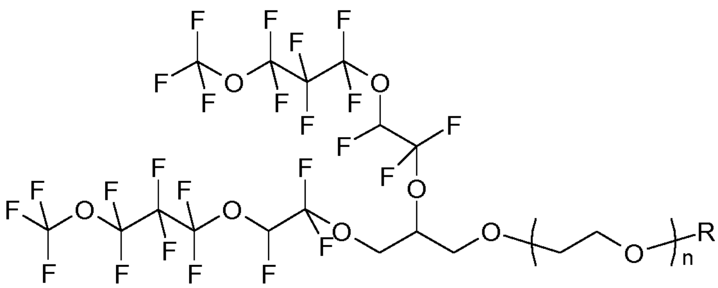

n~24 and R=H or methyl formula (VIII)

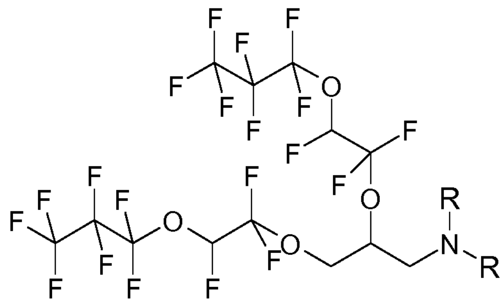

R=methyl or ethyl formula (IX)

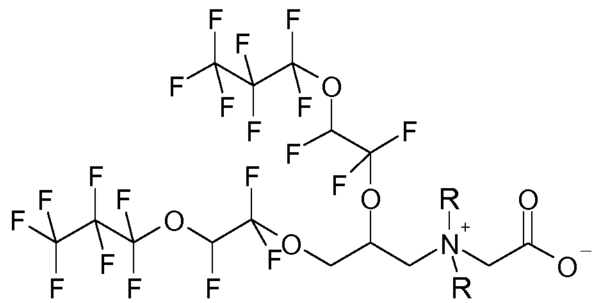

R=methyl or ethyl formula (X)

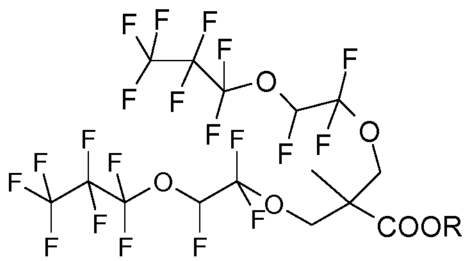

R=H, methyl or ethyl formula (XI)

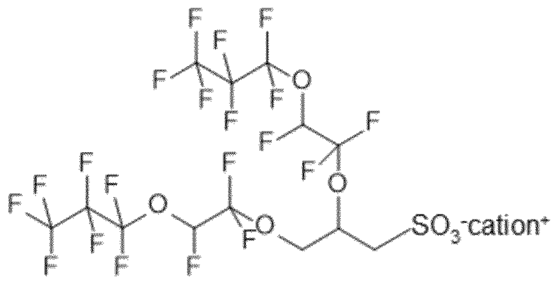

$cation^+ = Na^+, K^+$ or $NH_4^+$

formula (XII)

**12.** Use of compounds according to one or more of Claims 1 to 11 as additives in paints, coatings, printing inks, protective

coatings, speciality coatings in electronic or optical applications, photoresists, top antireflective coatings or bottom antireflective coatings, developer solutions and wash solutions and photoresists for photolithographic processes, cosmetic products, agrochemicals, floor polishes, photographic coatings or coatings of optical elements.

**13.** Composition comprising a compound according to one or more of Claims 1 to 11 and a vehicle which is suitable for the respective application and optionally further specific active substances.

**14.** Composition according to Claim 13, **characterised in that** the composition encompasses paint and coating preparations, fire-extinguishing compositions, lubricants, washing and cleaning compositions, de-icers, developer solutions and wash solutions and photoresists for photolithographic processes, cosmetic products, agrochemicals, floor polishes or hydrophobicising compositions for textile finishing or glass treatment.

**Revendications**

**1.** Composés de formules (I), (IIa) ou (IIb)

$$Z_n espaceurX_x \qquad (I)$$

où

$Z = R_f-CHF-CF_2-Y-(CH_2)_m-$ ou
$R_f-O-CHF-CF_2-Y-(CH_2)_m-$ ou
$R_f-O-(CF_2)_{1-4}-CHF-CF_2-Y-(CH_2)_m-$ ou
$R_f-O-(CF_2)_{1-4}-O-CHF-CF_2-Y-(CH_2)_m-$ ou
$R_f-O-(CF(CF_3)-CF_2)_{1-4}-CHF-CF_2-Y-(CH_2)_m-$ ou
$R_f-O-(CF(CF_3)-CF_2)_{1-4}-O-CHF-CF_2-Y-(CH_2)_m-$ ou
$R_f-O-(CF_2-O)_{1-4}-CHF-CF_2-Y-(CH_2)_m-$ ou
$R_f-O-(CF_2-CF_2-O)_{1-4}-CHF-CF_2-Y-(CH_2)_m-$ ou
$R_f-O-(CF_2-O)_{1-4}-(CF_2-CF_2-O)_{1-4}-CHF-CF_2-Y-(CH_2)_m-$,
n = 2 ou 3,
Rf = $CF_3-$, $CF_3-CF_2-$ ou $CF_3-CF_2-CF_2-$,
Y = O ou S,
m = 0 ou 1,
espaceur = un motif hydrocarboné saturé, ramifié ou non ramifié, où un ou plusieurs atomes de C non adjacents peuvent être remplacés par O ou N,
X est l'un des groupements $-SO_3^-$, $-OSO_3^-$, $-COO^-$, $-PO_3^{2-}$ ou $OPO_3^{2-}$, polyéthylène glycol ou polypropylène glycol, $-CH(OH)-CH_2-NH-sach$, $-Y-(CH_2-CH_2-O)_v-R^4$, bétaïnes ou sulfobétaïnes, où « sach » = divers sucres et Y = S, O ou NH, de préférence O, $R^4$ = H ou alkyle, de préférence H ou $CH_3$, et v = 1=100, de préférence 1-30,
x = 1,

$$[F(CF_2)_{n'}(O)_oCHF-CF_2-Y-(CH_2)_m]_2espaceur-X \text{ formule} \qquad (IIa)$$

$$[F(CF_2)_{n'}(O)_o-(CF_2)_{1-4}-O-CHF-CF_2-Y-(CH_2)_m]_2espaceur-X \text{ formule} \qquad (IIb)$$

où n' = 1-6, m = 0 ou 1, o = 0 ou 1,
Y = O ou S, et le groupement (espaceur-X) = $CR^5(CH_2)_{n''}OH$, $CR^5(CH_2)_{n''}SH$, $CR^5(CH_2)_{n''}COOH$, $CR^5(CH_2)_{n''}SO_3H$, $CR^5(CH_2)_{n''}NH_2$, $CR^5(CH_2)_{n''}NR'_2$, $CR^5(CH_2)_{n''}N^+(CH_3)_3Cl^-$, $CR^5(CH_2)_{n''}NR'_2-CH_2-COO^-$, $CR^5(CH_2)_{n''}O(CHR^a-CHR^bO)_nR''$, $CR^5(CH_2)_{n''}S(CHR^a-CHR^bO)_nR''$ ou $CR^5(CH_2)_{n''}NH(CHR^a-CHR^bO)_nR''$, n'' = 0 ou 1, n = 1-30, et $R^5$, R', R'', $R^a$ et $R^b$ indépendamment les uns des autres = H ou alkyle,
et où tous les indices sont choisis de sorte qu'aucune liaison O-O ne soit présente.

**2.** Composés selon la revendication 1, **caractérisés en ce que** l'espaceur est un groupement alkylène saturé, ramifié ou non ramifié, où un ou plusieurs atomes de C non adjacents peuvent être remplacés par O ou N.

**3.** Composés selon l'une ou plusieurs parmi les revendications 1 à 2, **caractérisés en ce que**
Z est égal à :

$R_f$-CHF-$CF_2$-Y-$(CH_2)_m$- ou
$R_f$-O-CHF-$CF_2$-Y-$(CH_2)_m$- ou
$R_f$-O-$(CF_2)_{1-4}$-CHF-$CF_2$-Y-$(CH_2)_m$- ou
$R_f$-O-$(CF_2)_{1-4}$-O-CHF-$CF_2$-Y-$(CH_{2)m}$-, où m = 0 ou 1.

**4.** Composés selon l'une ou plusieurs parmi les revendications 1 à 3, **caractérisés en ce que** Z est égal à :
$R_f$-O-CHF-$CF_2$-Y-$(CH_2)_m$- ou $R_f$-O-$(CF_2)_{1-4}$-O-CHF-$CF_2$-Y-$(CH_{2)m}$-, où m = 0 ou 1.

**5.** Composés selon l'une ou plusieurs parmi les revendications 1 à 4, **caractérisés en ce que** Rf = $CF_3$- ou $CF_3$-$CF_2$-$CF_2$-.

**6.** Composés selon l'une ou plusieurs parmi les revendications 1 à 5, **caractérisés en ce que** le groupement (espaceur-X) dans la formule (I) revêt l'une des significations suivantes :

$CR^5(CH_2)_{n''}OH$, $CR^5(CH_2)_{n''}SH$, $CR^5(CH_2)_{n''}COOH$, $CR^5(CH_2)_{n''}SO_3H$, $CR^5(CH_2)_{n''}NH_2$, $CR^5(CH_2)_{n''}NR'_2$, $CR^5(CH_2)_{n''}N^+(CH_3)_3Cl^-$, $CR^5(CH_2)_{n''}NR'_2$-$CH_2$-$COO^-$, $CR^5(CH_2)_{n''}O(CHR^a$-$CHR^bO)_nR''$, $CR^5(CH_2)_{n''}S(CHR^a$-$CHR^bO)_nR''$ ou $CR^5(CH_2)_{n''}NH(CHR^a$-$CHR^bO)_nR''$, où n" = 0 ou 1, n = 1-30 et $R^5$, R', R", $R^a$ et $R^b$ indépendamment les uns des autres = H ou alkyle.

**7.** Composés selon l'une ou plusieurs parmi les revendications 1 à 6, **caractérisés en ce qu'**ils répondent à la formule (IIa) ou (IIb) :

$[F(CF_2)_{n'}(O)_oCHF$-$CF_2$-Y-$(CH_2)_m]_2$espaceur-X formule (IIa)

$[F(CF_2)_{n'}(O)_o$-$(CF_2)_{1-4}$-O-CHF-$CF_2$-Y-$(CH_2)_m]_2$espaceur-X formule (IIb)

où n' = 1-6, m = 0 ou 1, o = 0 ou 1,
Y = O, et le groupement (espaceur-X) = $CR^5(CH_2)_{n''}OH$, $CR^5(CH_2)_{n''}SH$, $CR^5(CH_2)_{n''}COOH$, $CR^5(CH_2)_{n''}SO_3H$, $CR^5(CH_2)_{n''}NH_2$, $CR^5(CH_2)_{n''}NR'_2$, $CR^5(CH_2)_{n''}N^+(CH_3)_3Cl^-$, $CR^5(CH_2)_{n''}NR'_2$-$CH_2$-$COO^-$, $CR^5(CH_2)_{n''}O(CHR^a$-$CHR^bO)_nR''$, $CR^5(CH_2)_{n''}S(CHR^a$-$CHR^bO)_nR''$ ou $CR^5(CH_2)_{n''}NH(CHR^a$-$CHR^bO)_nR''$, n" = 0 ou 1,
n = 1-30, et $R^5$, R', R", $R^a$ et $R^b$ indépendamment les uns des autres = H ou alkyle.

**8.** Composés selon l'une ou plusieurs parmi les revendications 1 à 7, **caractérisés en ce que** n' = 1-3, m = 0 ou 1, o = 1, Y = O, et R', R", $R^a$ et $R^b$ indépendamment les uns des autres = H ou C1-C4 alkyle.

**9.** Composés selon l'une ou plusieurs parmi les revendications 1 à 8, **caractérisés en ce qu'**ils répondent à la formule (III), (III'), (IV) ou (IV') :

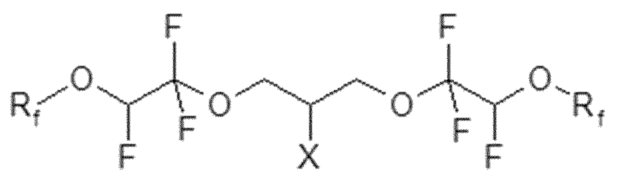
formule (III)

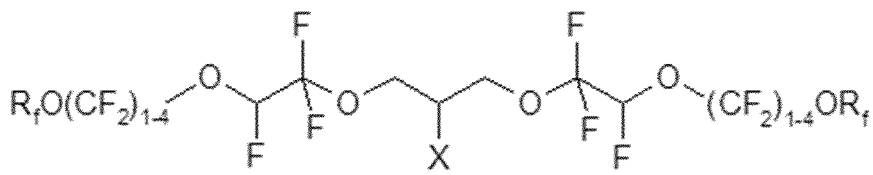

formule (III')

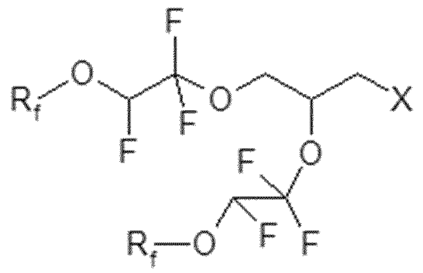

formule (IV)

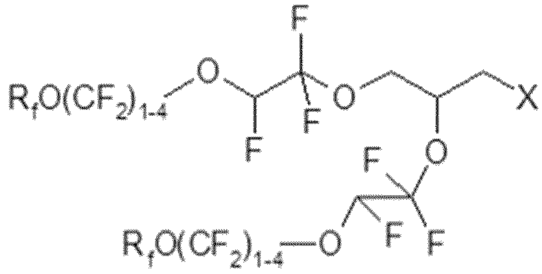

formule (IV')

où $R_f$ = alkyle fluoré, linéaire ou ramifié, de préférence C1-C4-alkyle perfluoré, et X est un groupement hydrophile.

**10.** Composés selon l'une ou plusieurs parmi les revendications 1 à 9, **caractérisés en ce qu'**ils répondent aux formules (IIIa), (III'a), (IIIb), (III'b), (IVa), (IV'a), (IVb) (IV'ba), (IVc) ou (IV'c) :

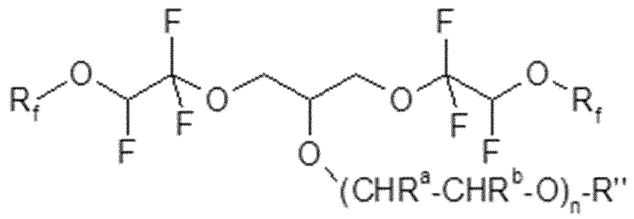

formule (IIIa)

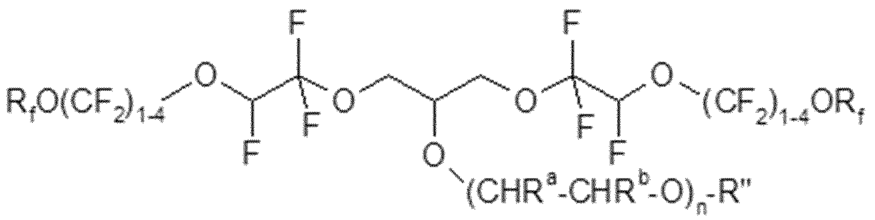

formule (III'a)

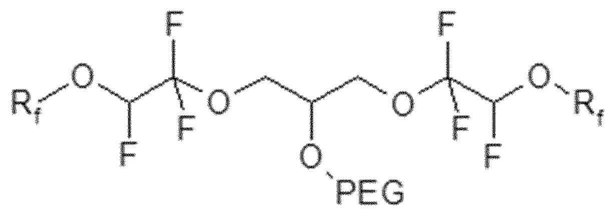

formule (IIIb)

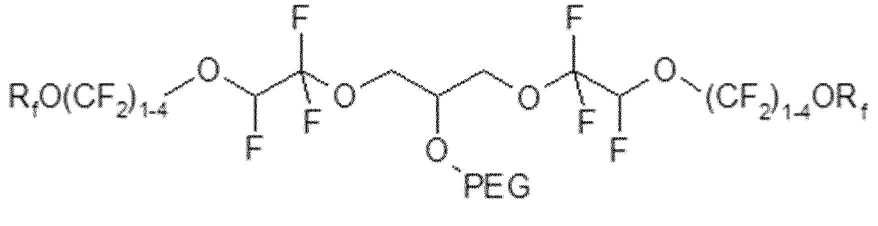

formule (III'b)

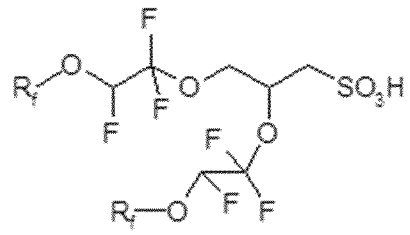

formule (IVa)

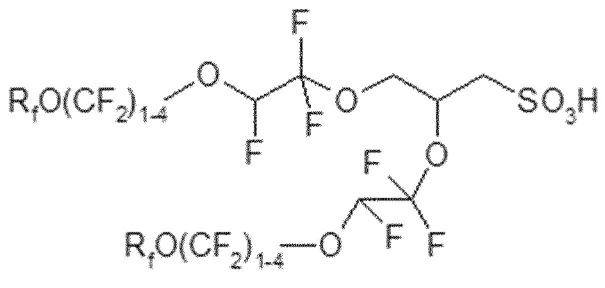

formule (IV'a)

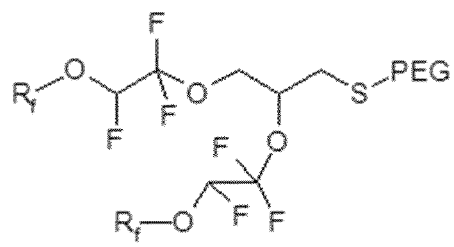

formule (IVb)

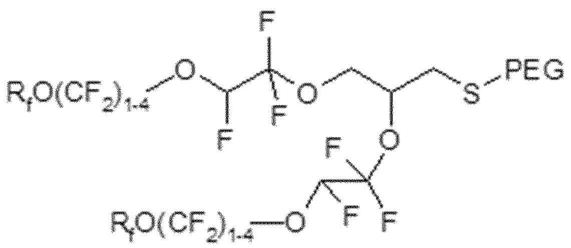

formule (IV'b)

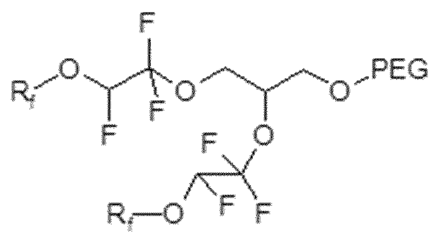

formule (IVc)

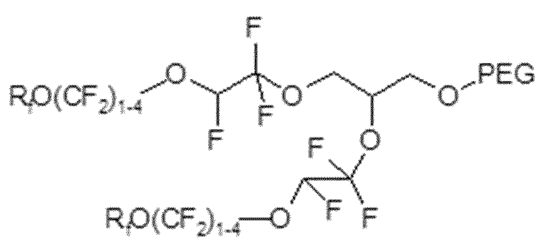

formule (IV'c)

où $R_f$ = alkyle fluoré, linéaire ou ramifié, de préférence C1-C4-alkyle perfluoré, et PEG représente polyéthylène glycol, polypropylène glycol, polyéthylène glycol alkyléther ou polypropylène glycol alkyléther.

**11.** Composés selon l'une ou plusieurs parmi les revendications 1 à 10, **caractérisés en ce qu'**ils répondent à l'une des formules (V) à (XII) :

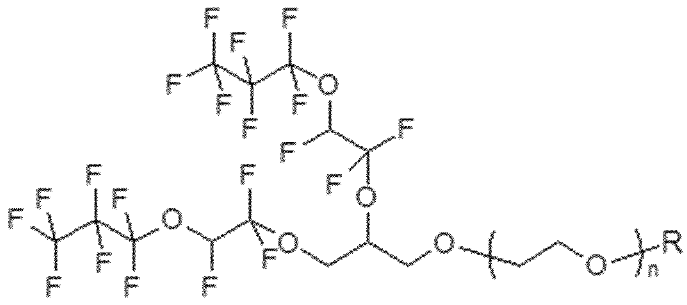

n~6 et R = H ou méthyle

formule (V)

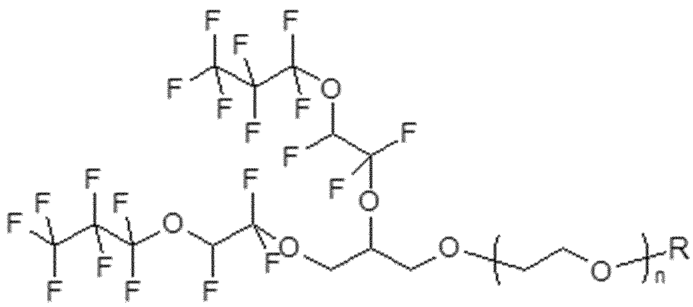

n~12 et R = H ou méthyle

formule (VI)

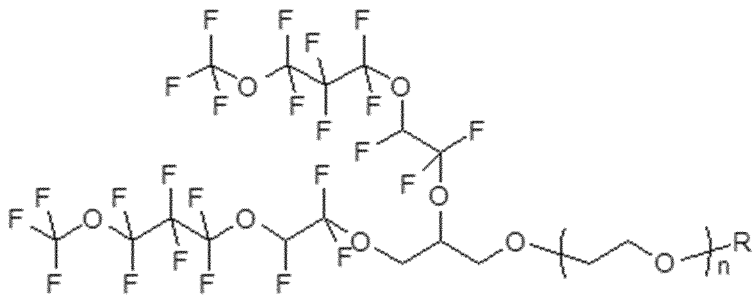

n~12 et R = H ou méthyle

formule (VII)

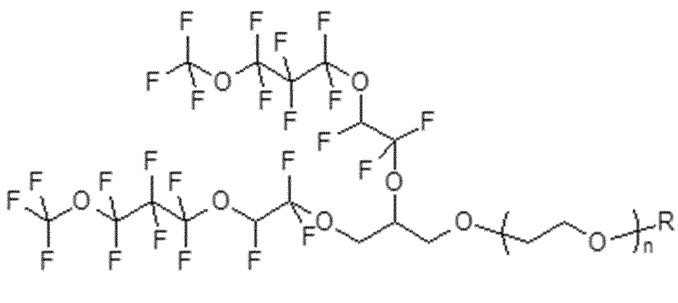

n~24 et R = H ou méthyle

formule (VIII)

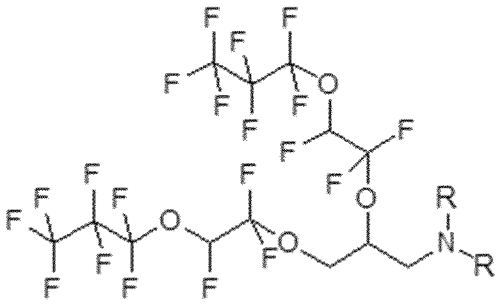

R = méthyle ou éthyle

formule (IX)

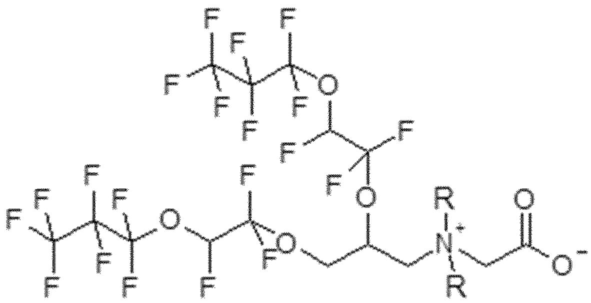

R = méthyle ou éthyle

formule (X)

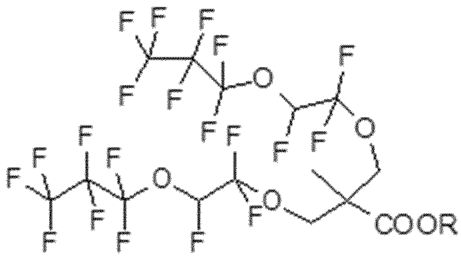

R = H, méthyle ou éthyle

formule (XI)

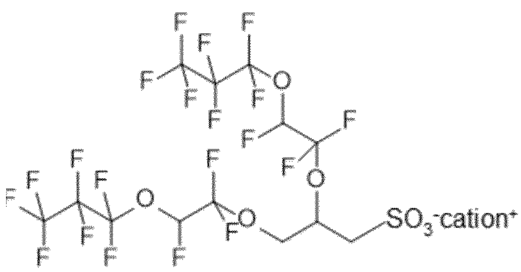

$cation^+ = Na^+, K^+$ ou $NH_4^+$

formule (XII)

**12.** Utilisation de composés selon l'une ou plusieurs parmi les revendications 1 à 11, comme additifs dans les peintures, les revêtements, les encres d'impression, les revêtements protecteurs, les revêtements spéciaux dans les applications électroniques ou optiques, les résines photosensibles, les revêtements antireflets supérieurs ou les revêtements antireflets inférieurs, les solutions de révélateur et les solutions de rinçage et les résines photosensibles pour les procédés photo-lithographiques, les produits cosmétiques, les substances agrochimiques, les encaustiques, les revêtements photographiques ou les revêtements pour les éléments optiques.

**13.** Composition comprenant un composé selon l'une ou plusieurs parmi les revendications 1 à 11, et un véhicule qui est convenable pour l'application respective, et éventuellement d'autres substances actives spécifiques.

**14.** Composition selon la revendication 13, **caractérisée en ce que** la composition englobe les préparations de peintures et de revêtements, les compositions d'extinction d'incendies, les lubrifiants, les compositions de lavage et de nettoyage, les dégivreurs, les solutions de révélateur et les solutions de rinçage et les résines photosensibles pour les procédés photo-lithographiques, les produits cosmétiques, les substances agrochimiques, les encaustiques ou les compositions d'hydrophobie pour la finition des textiles ou le traitement du verre.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 03010128 A
- JP 2001133984 A
- JP 9111286 A
- WO 2006072401 A
- WO 2010003567 A
- US 7635789 B
- US 20080093582 A
- JP 2004018394 A
- WO 2010002623 A
- US 7737307 B
- EP 1522536 A
- WO 2010002622 A
- WO 2009020907 A
- US 4968599 A
- US 4988610 A
- US 6890608 B
- WO 2009149807 A
- WO 2010149262 A
- WO 2011082770 A
- WO 2012084118 A
- DD 373314


### In der Beschreibung aufgeführte Nicht-Patentliteratur


- **G. L. KENNEDY, JR. ; J. L. BUTENHOFF ; G. W. OLSEN ; J. C. O'CONNOR ; A. M. SEACAT ; R. G. PERKINS ; L. B. BIEGEL ; S. R. MURPHY ; D. G. FARRAR.** *Critical Reviews in Toxicology,* 2004, vol. 34, 351-384
- **A.R. PITT et al.** *Colloids and Surfaces A: Physicochemical and Engineering Aspects,* 1996, vol. 114, 321-335
- **A.R. PITT.** *Progr. Colloid Polym. Sci,* 1997, vol. 103, 307-317
- **Z.-T. LIU et al.** *Ind. Eng. Chem. Res.,* 2007, vol. 46, 22-28